(19) Europäisches Patentamt
European Patent Office
Office européen des brevets

(11) EP 3 527 558 A1

(12) EUROPEAN PATENT APPLICATION

(43) Date of publication:
**21.08.2019 Bulletin 2019/34**

(51) Int Cl.:
*C07D 251/24* (2006.01) *H01L 51/00* (2006.01)

(21) Application number: **18157180.3**

(22) Date of filing: **16.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Novaled GmbH**
**01307 Dresden (DE)**

(72) Inventors:
- **FREY, Julien**
  **01307 Dresden (DE)**
- **GALAN, Elena**
  **01307 Dresden (DE)**

(74) Representative: **Michalski Hüttermann & Partner Patentanwälte mbB**
**Speditionstraße 21**
**40221 Düsseldorf (DE)**

(54) **N-HETEROARYLENE COMPOUNDS**

(57) The present invention relates to a compound according to formula 1:

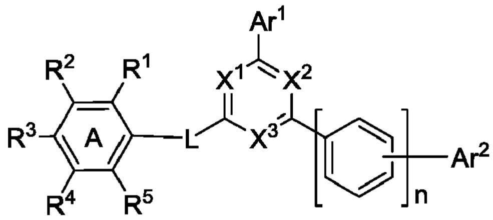

1,

suitable for use as a layer material for electronic devices, and to an organic semiconductor layer comprising at least one compound according to formula 1, as well as to an organic electronic device comprising at least one organic semiconductor layer, and a method of manufacturing the same.

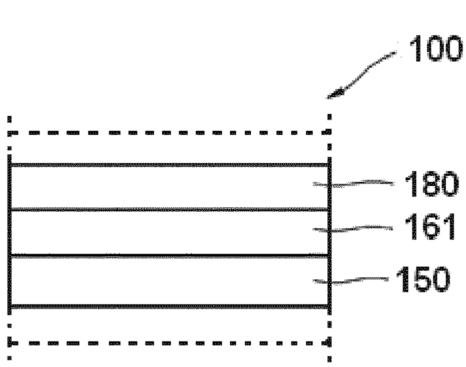


FIG. 1



Printed by Jouve, 75001 PARIS (FR)

## Description

### Technical Field

The present invention relates to N-heteroarylene compounds, in particular to N-heteroarylene compounds substituted with bulky groups, suitable for use as a layer material for electronic devices, and relates to an organic semiconductor layer comprising at least one compound thereof, as well as to an organic electronic device comprising at least one organic semiconductor layer, and a method of manufacturing the same.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic material being capable of increasing electron mobility and simultaneously increasing electrochemical stability is needed so that the organic electronic device, such as an organic light emitting diode, may be applied to a large-size flat panel display.

Further, development of an organic material being capable to have an extended life span at higher current density and thereby at higher brightness is needed.

There remains a need to improve performance of organic semiconductor layers, organic semiconductor materials, as well as organic electronic devices thereof, in particular to achieve increased lifetime at higher current density and have a higher efficiency through improving the characteristics of the compounds comprised therein.

There is a need for alternative organic semiconductor materials and organic semiconductor layers as well as organic electronic devices having increased lifetime at higher current density, and/or improved efficiency at low operating voltage.

In particular there is a need for alternative compounds having increased lifetime at higher current density as well as improved efficiency, and at the same time keeping the operating voltage and thereby the power consumption low to deliver long battery life for example mobile electronic devices.

### DISCLOSURE

An aspect of the present invention provides a compound of formula 1:

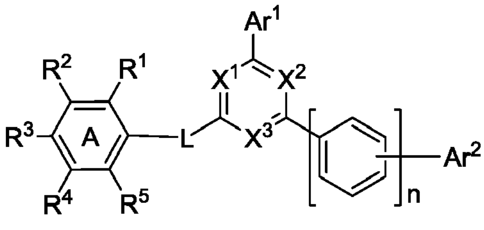 (1),

wherein

| | |
|---|---|
| $X^1$ to $X^3$ | are selected from N or C, with the proviso that at least two of $X^1$ to $X^3$ are selected from N; |
| L | is phenylene; |
| $R^1$ to $R^5$ | are independently selected from H, phenyl or pyridyl, with the proviso that at least 2 of $R^1$ to $R^5$ is phenyl or pyridyl; |
| $Ar^1$ | is selected from substituted or unsubstituted $C_6$ to $C_{18}$ aryl or substituted or unsubstituted $C_2$ to $C_{20}$ heteroaryl; |

| | |
|---|---|
| $Ar^2$ | is selected from substituted or unsubstituted aryl group comprising at least 3 phenyl rings; and |
| n | is selected from 0, 1 or 2; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

If not otherwise stated H can represent hydrogen or deuterium.

According to one embodiment of the compound of formula 1:

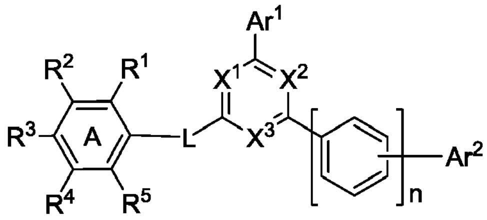

(1),

wherein

| | |
|---|---|
| $X^1$ to $X^3$ | are selected from N or C, with the proviso that at least two of $X^1$ to $X^3$ are selected from N; |
| L | is phenylene; |
| $R^1$ to $R^5$ | are independently selected from H, phenyl or pyridyl, with the proviso that at least 2 of $R^1$ to $R^5$ is phenyl or pyridyl; |
| $Ar^1$ | is selected from substituted or unsubstituted $C_6$ to $C_{18}$ aryl or substituted or unsubstituted $C_2$ to $C_{20}$ heteroaryl; |
| $Ar^2$ | is selected from substituted or unsubstituted aryl group comprising at least 3 phenyl rings; and |
| n | is selected from 0 or 1; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

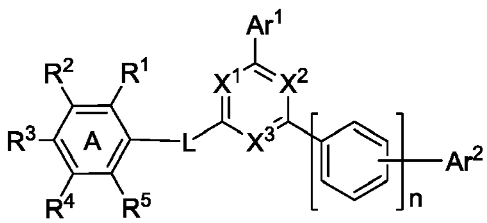

(1),

wherein

| | |
|---|---|
| $X^1$ to $X^3$ | are N; |
| L | is phenylene; |
| $R^1$ to $R^5$ | are independently selected from H, phenyl or pyridyl, with the proviso that at least 2 of $R^1$ to $R^5$ is phenyl or pyridyl; |
| $Ar^1$ | is selected from substituted or unsubstituted $C_6$ to $C_{18}$ aryl or substituted or unsubstituted $C_2$ to $C_{20}$ heteroaryl; |
| $Ar^2$ | is selected from substituted or unsubstituted aryl group comprising at least 3 phenyl rings; and |
| n | is selected from 0, 1 or 2; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

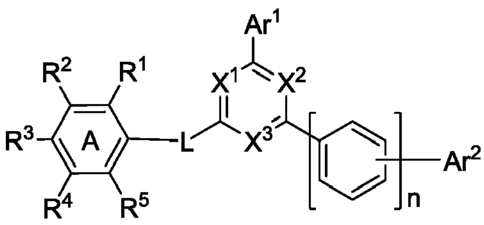

(1),

wherein

| | |
|---|---|
| $X^1$ to $X^3$ | are N; |
| L | is phenylene; |
| $R^1$ to $R^5$ | are independently selected from H, phenyl or pyridyl, with the proviso that at least 2 of $R^1$ to $R^5$ is phenyl or pyridyl; |
| $Ar^1$ | is selected from substituted or unsubstituted $C_6$ to $C_{18}$ aryl or substituted or unsubstituted $C_2$ to $C_{20}$ heteroaryl; |
| $Ar^2$ | is selected from substituted or unsubstituted aryl group comprising at least 3 phenyl rings; and |
| n | is selected from 0 or 1; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

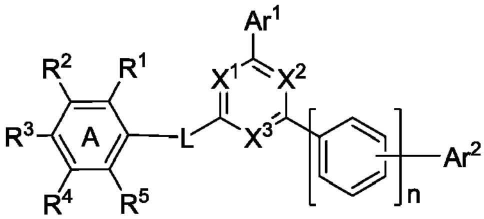

(1),

wherein

| | |
|---|---|
| $X^1$ to $X^3$ | are N; |
| L | is phenylene; |
| $R^1$ to $R^5$ | are independently selected from H, phenyl or pyridyl, with the proviso that at least 3 to 5 of $R^1$ to $R^5$ are phenyl or pyridyl, preferably at least 3 to 5 of $R^1$ to $R^5$ are phenyl; |
| $Ar^1$ | is selected from substituted or unsubstituted $C_6$ to $C_{18}$ aryl or substituted or unsubstituted $C_2$ to $C_{20}$ heteroaryl; |
| $Ar^2$ | is selected from substituted or unsubstituted aryl group comprising at least 3 phenyl rings; and |
| n | is selected from 0, 1 or 2; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

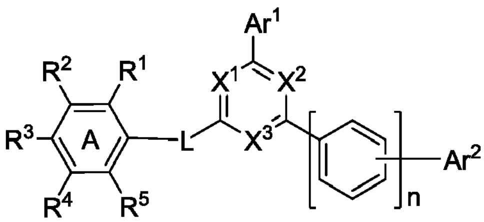

(1),

wherein

$X^1$ to $X^3$ are N;
L is phenylene;
$R^1$ to $R^5$ are independently selected from H, phenyl or pyridyl, with the proviso that at least 3 to 5 of $R^1$ to $R^5$ are phenyl or pyridyl, preferably at least 3 to 5 of $R^1$ to $R^5$ are phenyl;
$Ar^1$ is selected from substituted or unsubstituted $C_6$ to $C_{18}$ aryl or substituted or unsubstituted $C_2$ to $C_{20}$ heteroaryl;
$Ar^2$ is selected from substituted or unsubstituted aryl group comprising at least 3 phenyl rings; and
n is selected from 0 or 1;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

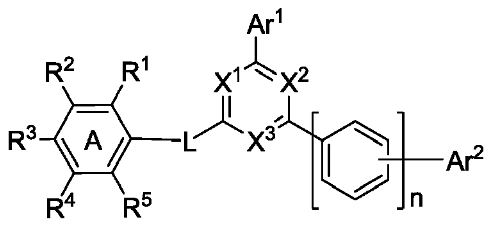
(1),

wherein

$X^1$ to $X^3$ are N;
L is phenylene;
$R^1$ to $R^5$ are independently selected from H, phenyl or pyridyl, with the proviso that at least 3 to 5 of $R^1$ to $R^5$ are phenyl or pyridyl, preferably at least 3 to 5 of $R^1$ to $R^5$ are phenyl;
$Ar^1$ is selected from substituted or unsubstituted $C_6$ to $C_{18}$ aryl or substituted or unsubstituted $C_2$ to $C_{20}$ heteroaryl;
$Ar^2$ is selected from substituted or unsubstituted aryl group comprising at least 4 phenyl rings to 12 phenyl rings, preferably 5 phenyl rings to 10 phenyl rings, and further preferred 7 phenyl rings to 9 phenyl rings; and
n is selected from 0, 1 or 2;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

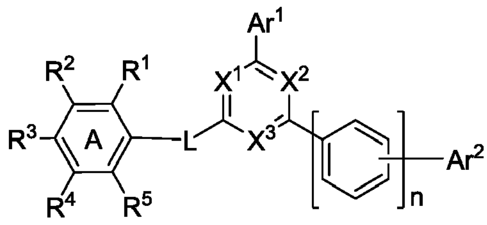
(1),

wherein

$X^1$ to $X^3$ are N;
L is phenylene;
$R^1$ to $R^5$ are independently selected from H, phenyl or pyridyl, with the proviso that at least 3 to 5 of $R^1$ to $R^5$ are phenyl or pyridyl, preferably at least 3 to 5 of $R^1$ to $R^5$ are phenyl;
$Ar^1$ is selected from substituted or unsubstituted $C_6$ to $C_{18}$ aryl or substituted or unsubstituted $C_2$ to $C_{20}$ heteroaryl;
$Ar^2$ is selected from substituted or unsubstituted aryl group comprising at least 4 phenyl rings to 12 phenyl rings, preferably 5 phenyl rings to 10 phenyl rings, and further preferred 7 phenyl rings to 9 phenyl rings; and
n is selected from 0 or 1;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

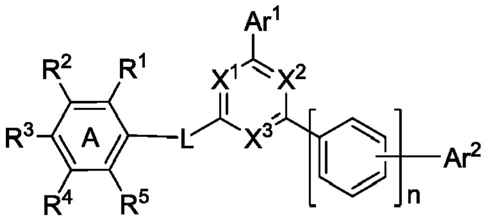

(1),

wherein

| | |
|---|---|
| $X^1$ to $X^3$ | are N; |
| L | is phenylene; |
| $R^1$ to $R^5$ | are independently selected from H, phenyl or pyridyl, with the proviso that at least 3 to 5 of $R^1$ to $R^5$ are phenyl or pyridyl, preferably at least 3 to 5 of $R^1$ to $R^5$ are phenyl; |
| $Ar^1$ | is selected from substituted or unsubstituted $C_6$ to $C_{18}$ aryl, wherein the substituents of the substituted $C_6$ to $C_{18}$ aryl are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl; and preferably $Ar^1$ is selected from unsubstituted $C_6$ to $C_{18}$ aryl; |
| $Ar^2$ | is selected from substituted or unsubstituted aryl group comprising at least 4 phenyl rings to 12 phenyl rings, preferably 5 phenyl rings to 10 phenyl rings, and further preferred 7 phenyl rings to 9 phenyl rings; and |
| n | is selected from 0, 1 or 2; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to one embodiment of the compound of formula 1:

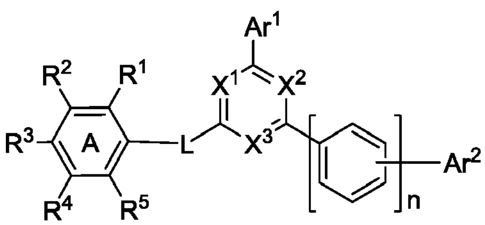

(1),

wherein

| | |
|---|---|
| $X^1$ to $X^3$ | are N; |
| L | is phenylene; |
| $R^1$ to $R^5$ | are independently selected from H, phenyl or pyridyl, with the proviso that at least 3 to 5 of $R^1$ to $R^5$ are phenyl or pyridyl, preferably at least 3 to 5 of $R^1$ to $R^5$ are phenyl; |
| $Ar^1$ | is selected from substituted or unsubstituted $C_6$ to $C_{18}$ aryl, wherein the substituents of the substituted $C_6$ to $C_{18}$ aryl are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl; and preferably $Ar^1$ is selected from unsubstituted $C_6$ to $C_{18}$ aryl; |
| $Ar^2$ | is selected from substituted or unsubstituted aryl group comprising at least 4 phenyl rings to 12 phenyl rings, preferably 5 phenyl rings to 10 phenyl rings, and further preferred 7 phenyl rings to 9 phenyl rings; and |
| n | is selected from 0 or 1; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to another embodiment of the compound of formula 1, wherein about two X can be N.

According to another embodiment of the compound of formula 1, wherein about three X can be N.

According to another embodiment of the compound of formula 1, wherein about two X can be N.

According to another embodiment of the compound of formula 1, wherein $X^1$ and $X^2$ are N and $X^3$ is C.

According to another embodiment of the compound of formula 1, wherein $X^2$ and $X^3$ are N and $X^1$ is C.

According to another embodiment of the compound of formula 1, wherein $X^1$ and $X^3$ are N and $X^2$ is C.

According to another embodiment of the compound of formula 1, wherein at least 3 to 5 of $R^1$ to $R^5$ may be phenyl or pyridyl. According to another embodiment of the compound of formula 1, wherein 4 of $R^1$ to $R^5$ may be phenyl or pyridyl.

According to another embodiment of the compound of formula 1, wherein at least 3 to 5 of $R^1$ to $R^5$ may be pyridyl. According to another embodiment of the compound of formula 1, wherein 4 of $R^1$ to $R^5$ may be pyridyl.

According to another embodiment of the compound of formula 1, wherein at least 3 to 5 of $R^1$ to $R^5$ may be phenyl. According to another embodiment of the compound of formula 1, wherein 4 of $R^1$ to $R^5$ may be phenyl.

According to another embodiment of the compound of formula 1, wherein $Ar^2$ can be selected from substituted or unsubstituted aryl group comprising at least 4 phenyl rings to 12 phenyl rings, preferably 5 phenyl rings to 10 phenyl rings, and further preferred 7 phenyl rings to 9 phenyl rings.

According to another embodiment of the compound of formula 1, wherein $Ar^2$ may be selected from substituted or unsubstituted aryl group comprising at least 3 substituted or unsubstituted phenyl rings to 12 substituted or unsubstituted phenyl rings, preferably 4 substituted or unsubstituted phenyl rings to 10 substituted or unsubstituted phenyl rings, and further preferred 4 substituted or unsubstituted phenyl rings to 6 substituted or unsubstituted phenyl rings, wherein the substituents of the substituted aryl group are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl; and preferably $Ar^2$ is selected from unsubstituted $C_6$ to $C_{18}$ aryl.

According to another embodiment of the compound of formula 1, wherein $Ar^2$ can be selected from unsubstituted aryl group comprising at least 3 unsubstituted phenyl rings to 12 unsubstituted phenyl rings, preferably 4 unsubstituted phenyl rings to 10 unsubstituted phenyl rings, and further preferred 4 unsubstituted phenyl rings to 6 unsubstituted phenyl rings.

According to the present invention, the $Ar^2$ group may comprise at least three phenyl rings which are connected via one or more single bonds, via an ethylene group and/or are part of a fused ring system. If the at least three phenyl rings are part of a fused ring system, two or more phenyl rings may share at least two carbon atoms and/or two or more phenyl rings may be fused with 5-membered rings.

Preferably, $Ar^2$ comprises at least three phenyl rings which are connected via a single bond or are part of a fused ring system.

According to the present invention, the $Ar^2$ group may comprises at least three phenyl rings, wherein a first and/or a second phenyl ring are connected via a single bond to a third phenyl ring.

According to the present invention, the $Ar^2$ group may comprises at least three phenyl rings, wherein a first and/or a second phenyl ring are connected via an ethylene group to the third phenyl ring.

According to the present invention, the $Ar^2$ group may comprises at least three phenyl rings, wherein at least two or three of the first, second or third phenyl rings are part of a fused ring system.

According to the present invention, the $Ar^2$ group may comprises at least three phenyl rings, wherein at least the first, second and third phenyl ring are part of a fused ring system, wherein two or three of the phenyl rings may share at least two carbon atoms.

According to the present invention, the $Ar^2$ group may comprises at least three phenyl rings, wherein at least two of the first, second and third phenyl rings may be fused with a 5-membered ring.

According to the present invention, the $Ar^2$ group may comprises at least three phenyl rings, wherein at least two or three of the first, second and third phenyl rings may be fused each with a 5-membered ring, preferably two of the first, second and third phenyl rings may be fused to the same 5-membered ring.

According to another embodiment of the compound of formula 1, wherein for n = 1, formula 1 has the structure 2:

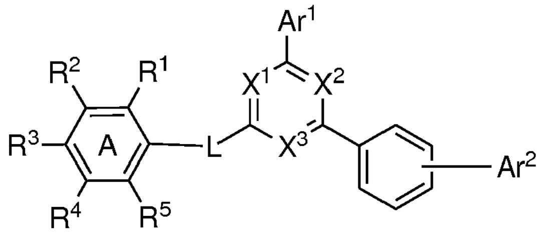

(2).

According to another embodiment of the compound of formula 1, wherein for n = 0, formula 1 has the structure 3:

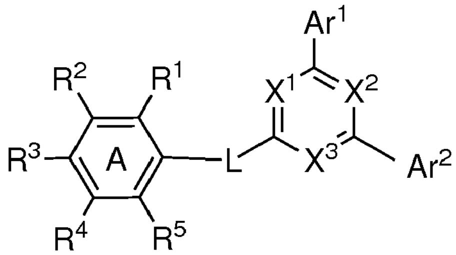

(3).

According to another embodiment of the compound of formula 1, wherein $Ar^1$ is selected from substituted or unsubstituted $C_6$ to $C_{18}$ aryl, wherein the substituents of the substituted $C_6$ to $C_{18}$ aryl are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of the compound of formula 1, wherein $Ar^1$ is selected from substituted $C_6$ to $C_{18}$ aryl, wherein the substituents of the substituted $C_6$ to $C_{18}$ aryl are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of the compound of formula 1, wherein $Ar^1$ can be preferably selected from unsubstituted $C_6$ to $C_{18}$ aryl.

According to another embodiment of the compound of formula 1, wherein $Ar^1$ is selected from substituted $C_2$ to $C_{20}$ hetero aryl, wherein the substituents of the substituted $C_2$ to $C_{20}$ heteroaryl are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl.

According to another embodiment of formula 1, wherein $Ar^1$ may be selected from pyridinyl, quinolinyl, quinazolinyl, dibenzofuranyl or dibenzothiophenyl.

According to another embodiment of the compound of formula 1, wherein $R^1$ and $R^2$ can be selected phenyl or pyridyl. According to another embodiment of the compound of formula 1, wherein $R^1$ and $R^2$ can be selected independantly phenyl or pyridyl. According to another embodiment of the compound of formula 1, wherein $R^1$ and $R^2$ can be selected phenyl. According to another embodiment of the compound of formula 1, wherein $R^1$ and $R^2$ can be selected pyridyl.

According to another embodiment of the compound of formula 1, wherein $R^1$ and $R^3$ can be selected phenyl or pyridyl. According to another embodiment of the compound of formula 1, wherein $R^1$ and $R^3$ can be selected independantly phenyl or pyridyl. According to another embodiment of the compound of formula 1, wherein $R^1$ and $R^3$ can be selected phenyl. According to another embodiment of the compound of formula 1, wherein $R^1$ and $R^3$ can be selected pyridyl.

According to another embodiment of the compound of formula 1, wherein $R^2$ and $R^3$ can be selected phenyl or pyridyl. According to another embodiment of the compound of formula 1, wherein $R^2$ and $R^3$ can be selected independantly phenyl or pyridyl. According to another embodiment of the compound of formula 1, wherein $R^2$ and $R^3$ can be selected phenyl. According to another embodiment of the compound of formula 1, wherein $R^2$ and $R^3$ can be selected pyridyl.

According to another embodiment of the compound of formula 1, wherein $R^1$, $R^2$ and $R^3$ can be selected phenyl or pyridyl. According to another embodiment of the compound of formula 1, wherein $R^1$, $R^2$ and $R^3$ can be selected independantly phenyl or pyridyl. According to another embodiment of the compound of formula 1, wherein $R^1$, $R^2$ and

$R^3$ can be selected phenyl. According to another embodiment of the compound of formula 1, wherein $R^1$, $R^2$ and $R^3$ can be selected pyridyl.

According to another embodiment of the compound of formula 1, wherein $R^1$, $R^2$, $R^3$ and $R^4$ can be selected phenyl or pyridyl. According to another embodiment of the compound of formula 1, wherein $R^1$, $R^2$, $R^3$ and $R^4$ can be selected independantly phenyl or pyridyl. According to another embodiment of the compound of formula 1, wherein $R^1$, $R^2$, $R^3$ and $R^4$ can be selected phenyl. According to another embodiment of the compound of formula 1, wherein $R^1$, $R^2$, $R^3$ and $R^4$ can be selected pyridyl.

According to another embodiment of the compound of formula 1, wherein $R^1$ and/or $R^3$ can be preferably phenyl or pyridyl, further preferred $R^3$ can be phenyl or pyridyl, also preferred $R^1$ and $R^3$ can be phenyl or pyridyl.

According to another embodiment of the compound of formula 1, wherein
$R^1$ and $R^2$ can be phenyl or pyridyl, and $R^3$, $R^4$ and $R^5$ can be H, or
$R^1$ and $R^3$ can be phenyl or pyridyl, and $R^2$, $R^4$ and $R^5$ can be H, or
$R^2$ and $R^3$ can be phenyl or pyridyl, and $R^1$, $R^4$ and $R^5$ can be H, or
$R^1$, $R^2$ and $R^3$ can be phenyl or pyridyl and $R^4$ and $R^5$ can be H, or
$R^2$, $R^3$ and $R^4$ can be phenyl or pyridyl and $R^1$ and $R^5$ can be H, or
R1, $R^2$, $R^3$ and $R^4$ can be phenyl or pyridyl and $R^5$ is H, or
R1, $R^2$, $R^3$, $R^4$ and $R^5$ can be phenyl or pyridyl.

According to another embodiment of the compound of formula 1, wherein $Ar^1$ may be selected from substituted or unsubstituted $C_6$ to $C_{12}$ aryl, wherein

the substituents of the substituted $C_6$ to $C_{12}$ aryl and substituted $C_3$ to $C_{36}$ heteroaryl may be selected from $C_1$ to $C_{12}$ alkyl, $C_1$ to $C_{12}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{24}$ aryl, $C_3$ to $C_{25}$ heteroaryl, D, F or CN, preferably from $C_1$ to $C_{12}$ alkyl.

According to another embodiment of the compound of formula 1, wherein
$Ar^1$ may be selected from unsubstituted $C_6$ to $C_{12}$ aryl, preferably a $C_6$ aryl.

According to another embodiment of the compound of formula 1, wherein
$Ar^2$ may be independently selected from B1 to B19:

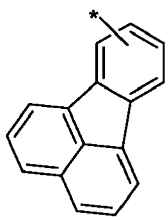
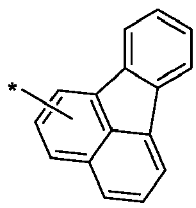
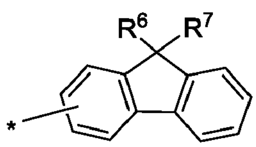
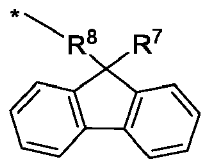

(B1), (B2), (B3), (B4),

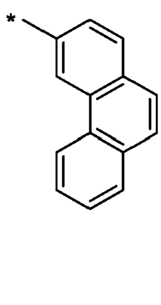
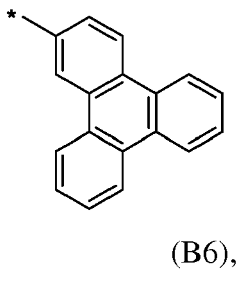

(B5), (B6),

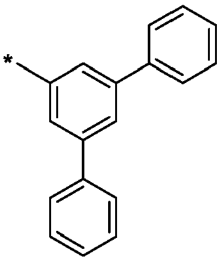 (B7), 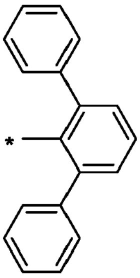 (B8), 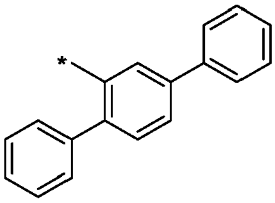 (B9), 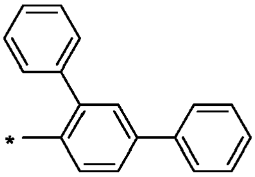 (B10),

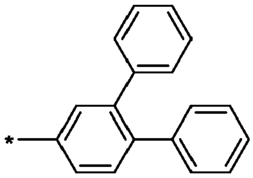 (B11),

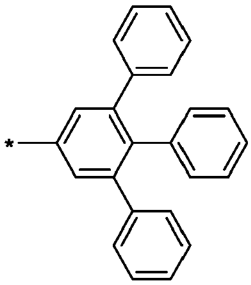 (B12), 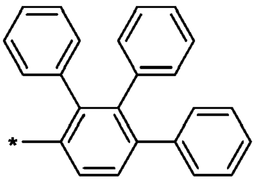 (B13), 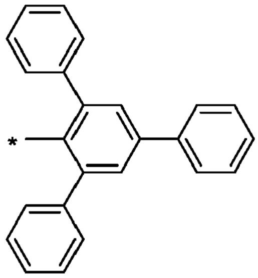 (B14),

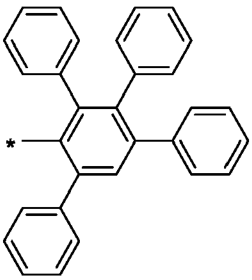 (B15), 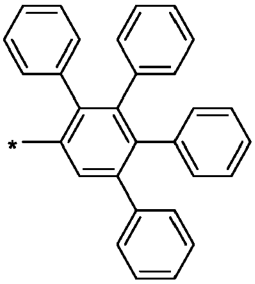 (B16), 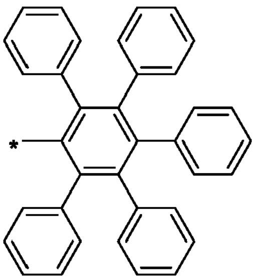 (B17),

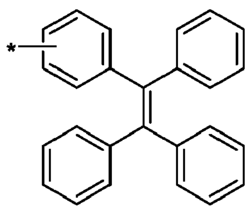 (B18), 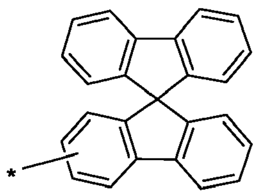 (B19);

wherein

$R^6$ and $R^7$ can be independently selected from H, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy or $C_6$ to $C_{24}$ aryl;

$R^8$ is selected from $C_6$ to $C_{18}$ arylene.

The asterix "*" in B1 to B19 denoting the bonding position, at which the substituents B1 to B19 of $Ar^2$ bonds in the compound according to formula 1.

In another embodiment, $Ar^2$ may be selected from B1 to B6, B7 to B11, B12 to B17, and/or B18 to B19, preferably from B1 to B6, B7 to B11 and/or B12 to B17; or further preferred from B7 to B11 and/or B12 to B17.

According to another embodiment of the compound of formula 1, wherein $R^6$ and $R^7$ can be independently selected from H, $C_1$ to $C_6$ alkyl, partially or perfluorinated $C_1$ to $C_6$ alkyl, partially or perdeuterated $C_1$ to $C_6$ alkyl, $C_1$ to $C_6$ alkoxy, $C_3$ to $C_6$ branched alkyl, $C_3$ to C6 cyclic alkyl, $C_3$ to $C_6$ branched alkoxy, $C_3$ to $C_6$ cyclic alkoxy or $C_6$ to $C_{18}$ aryl; $R^8$ can be selected from $C_6$ to $C_{12}$ arylene.

According to another embodiment of the compound of formula 1, wherein $R^6$ and $R^7$ can be independently selected from H or $C_6$ to $C_{18}$ aryl; $R^8$ can be selected from $C_6$ to $C_{12}$ arylene.

According to another embodiment of the compound of formula 1, wherein $R^6$ and $R^7$ can be independently selected from H; $R^8$ can be selected from $C_6$ to $C_{12}$ arylene.

According to another embodiment of the compound of formula 1, wherein $R^6$ and $R^7$ can be independently selected from $C_6$ to $C_{18}$ aryl; $R^8$ can be selected from $C_6$ to $C_{18}$ arylene.

According to another embodiment of the compound of formula 1, wherein $R^6$ and $R^7$ can be independently selected from $C_6$ to $C_{12}$ aryl; $R^8$ can be selected from $C_6$ to $C_{12}$ arylene.

According to another embodiment of the compound of formula 1 may be independently selected from structures C1 to C4:

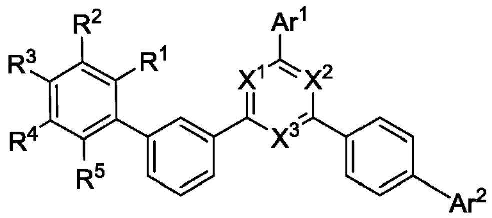

(C1),

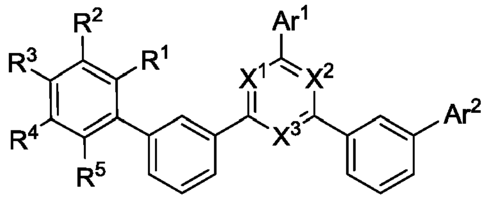

(C2),

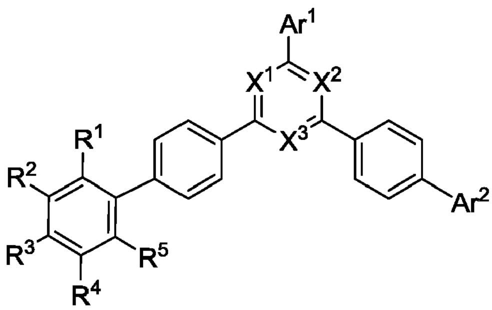

(C3),

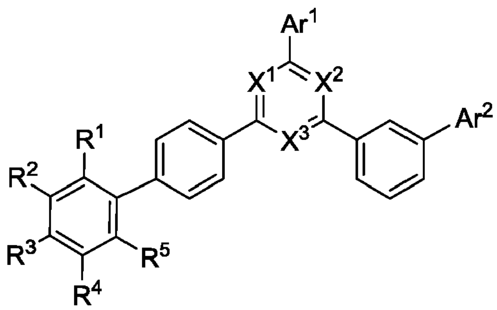

(C4),

wherein

$X^1$ to $X^3$ can be selected from N or C, with the proviso that at least two of $X^1$ to $X^3$ can be selected from N;

$R^1$ to $R^5$ can be independently selected from H, phenyl or pyridyl, with the proviso that at least two of $R^1$ to $R^5$ can be phenyl or pyridyl; and at least two of $R^1$ to $R^5$ that can be phenyl or pyridyl;

$Ar^1$ is selected from substituted or unsubstituted $C_6$ to $C_{18}$ aryl or substituted or unsubstituted $C_2$ to $C_{20}$ heteroaryl, preferably phenyl, wherein the substituents of the substituted aryl and substituted heteroaryl can be independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$

to $C_{16}$ alkoxy, $C_6$ to $C_{18}$ aryl, $C_3$ to $C_{25}$ heteroaryl, D, F or CN, preferably from $C_1$ to $C_{12}$ alkyl;

$Ar^2$ is selected from substituted or unsubstituted aryl group comprising at least three phenyl rings, preferably three to four phenyl rings;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to another embodiment of the compound of formula 1 may be independently selected from structures C1 to C4:

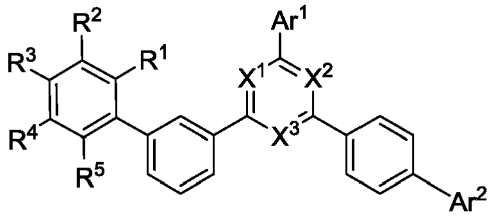

(C1),

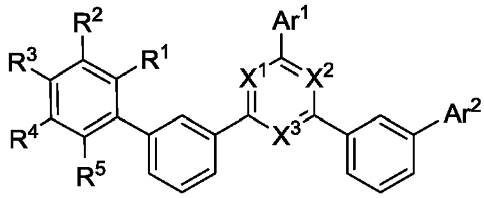

(C2),

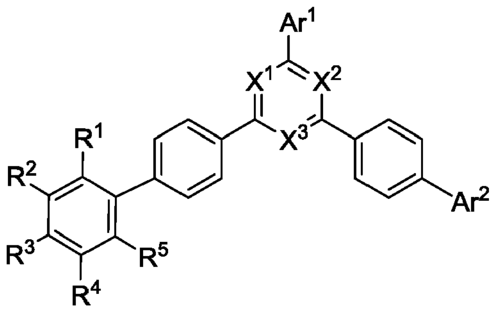

(C3),

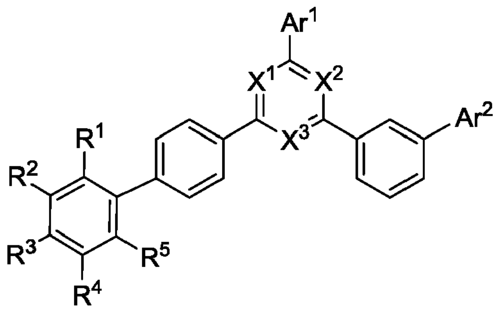

(C4),

wherein

$X^1$ to $X^3$ can be N;

$R^1$ to $R^5$ can be independently selected from H, phenyl or pyridyl, with the proviso that at least two of $R^1$ to $R^5$ can be phenyl or pyridyl; and at least two of $R^1$ to $R^5$ that can be phenyl or pyridyl;

$Ar^1$ is selected from substituted or unsubstituted $C_6$ to $C_{12}$ aryl or substituted or unsubstituted $C_2$ to $C_{18}$ heteroaryl, wherein the substituents of the substituted aryl and substituted heteroaryl can be independently selected from $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, $C_6$ to $C_{12}$ aryl, $C_3$ to $C_{11}$ heteroaryl, D, F or CN, preferably from $C_1$ to $C_6$ alkyl;

$Ar^2$ is selected from substituted or unsubstituted aryl group comprising at least three to four phenyl rings;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to another embodiment of the compound of formula 1 may be independently selected from structures C1 to C4:

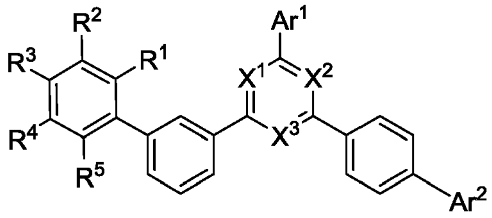

(C1),

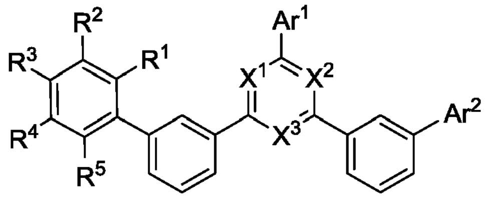

(C2),

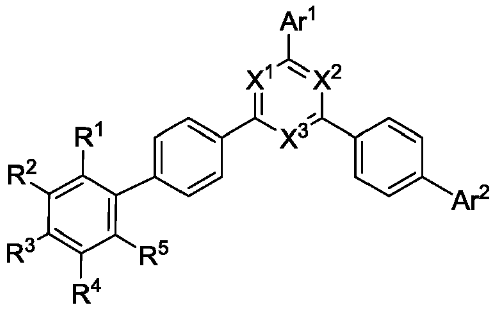

(C3),

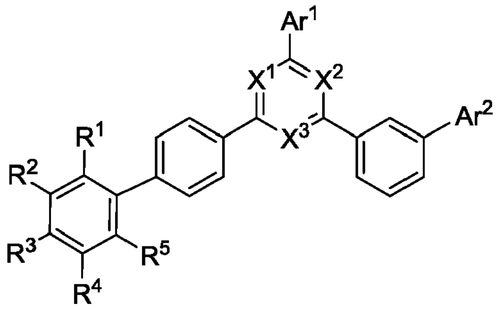

(C4),

wherein

$X^1$ to $X^3$ can be N;

$R^1$ to $R^5$ can be independently selected from H, phenyl, with the proviso that at least two of $R^1$ to $R^5$ can be phenyl; and at least two of $R^1$ to $R^5$ that can be phenyl;

$Ar^1$ is selected from unsubstituted $C_6$ to $C_{12}$ aryl or unsubstituted $C_2$ to $C_{18}$ heteroaryl;

$Ar^2$ is selected from unsubstituted aryl group comprising at least three phenyl rings, preferably three to four phenyl rings;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to another embodiment of the compound of formula 1 may be independently selected from structures C1 to C4:

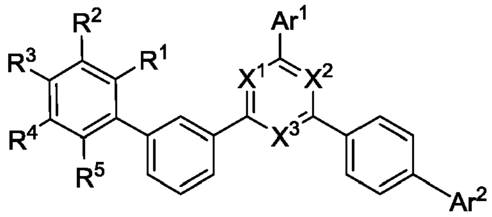

(C1),

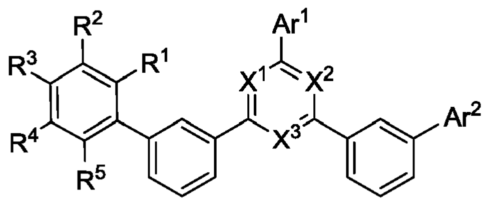

(C2),

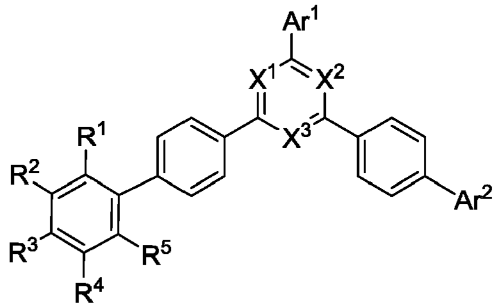

(C3),

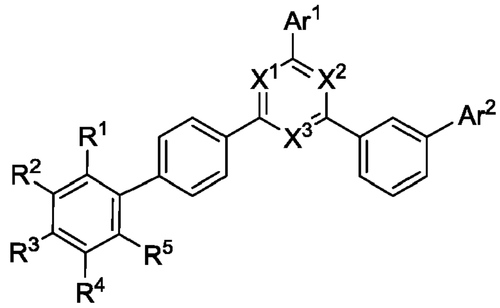

(C4),

wherein

| | |
|---|---|
| $X^1$ to $X^3$ | at least two can be N; |
| $R^1$ to $R^5$ | can be independently selected from H, pyridyl, with the proviso that at least two of $R^1$ to $R^5$ can be pyridyl; and at least two of $R^1$ to $R^5$ that can be pyridyl; |
| $Ar^1$ | is selected from unsubstituted $C_6$ to $C_{12}$ aryl or unsubstituted $C_2$ to $C_{18}$ heteroaryl; |
| $Ar^2$ | is selected from unsubstituted aryl group comprising at least three phenyl rings, preferably three to four phenyl rings; |

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

According to another embodiment of the compound of formula 1, wherein n = 0 or 1, preferably n = 0. According to another embodiment of the compound of formula 1, wherein n = 0. According to another embodiment of the compound of formula 1, wherein n = 1. According to another embodiment of the compound of formula 1, wherein n = 2.

According to another embodiment of the compound of formula 1, wherein

- n is 1; two of $X^1$, $X^2$ and $X^3$ are N and one is C, preferably $X^1$ and $X^2$ are N and $X^3$ is C; $Ar^1$ is phenyl; $R^1$, $R^2$, $R^3$, $R^4$, $R^5$ are independently selected from H and phenyl; and $Ar^2$ is selected from B1 to B19; with the proviso that at least 2 of $R^1$ to $R^5$ are phenyl, preferably at least 3 of $R^1$ to $R^5$ are phenyl, and in addition preferred 4 of $R^1$ to $R^5$ are phenyl and one is H; or

- n is 1; $X^1$, $X^2$ and $X^3$ are N; $Ar^1$ is phenyl; $R^1$, $R^2$, $R^3$, $R^4$, $R^5$ are independently selected from H and phenyl and $Ar^2$ is selected from B1 to B19; with the proviso that at least 2 of $R^1$ to $R^5$ are phenyl, preferably at least 3 of $R^1$ to $R^5$ are phenyl, and in addition preferred 4 of $R^1$ to $R^5$ are phenyl and one is H.

According to another embodiment of the present invention the compound of formula 1 can be selected from D1 to D108 as outlined in Table 1, wherein n is 1; two of $X^1$, $X^2$ and $X^3$ are N and one is C, preferably $X^1$ and $X^2$ are N and $X^3$ is C; $Ar^1$ is phenyl; and $R^1$, $R^2$, $R^3$, $R^4$, $R^5$ and $Ar^2$. Ph stands for phenyl.

Table 1

| Compounds D1 to D108 of formula 1, wherein n is 1; two of $X^1$, $X^2$ and $X^3$ are N and one is C, preferably $X^1$ and $X^2$ are N and $X^3$ is C; $Ar^1$ is phenyl | | | | | | |
|---|---|---|---|---|---|---|
| | $R^1$ | $R^2$ | $R^3$ | $R^4$ | $R^5$ | $Ar^2$ |
| (D1) | Ph | Ph | H | H | H | 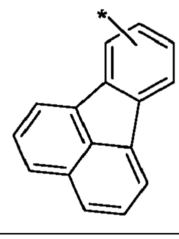 |

(continued)

| Compounds D1 to D108 of formula 1, wherein n is 1; two of $X^1$, $X^2$ and $X^3$ are N and one is C, preferably $X^1$ and $X^2$ are N and $X^3$ is C; $Ar^1$ is phenyl | | | | | | |
|---|---|---|---|---|---|---|
| | $R^1$ | $R^2$ | $R^3$ | $R^4$ | $R^5$ | $Ar^2$ |
| (D2) | Ph | Ph | H | H | H | 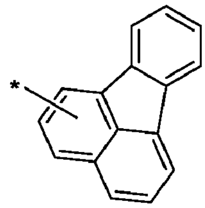 |
| (D3) | Ph | Ph | H | H | H | 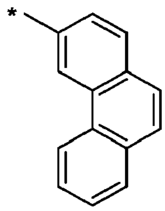 |
| | $R^1$ | $R^2$ | $R^3$ | $R^4$ | $R^5$ | $Ar^2$ |
| (D4) | Ph | Ph | H | H | H | 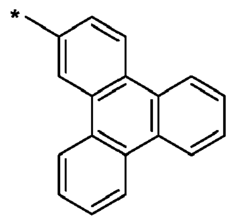 |
| (D5) | Ph | Ph | H | H | H | 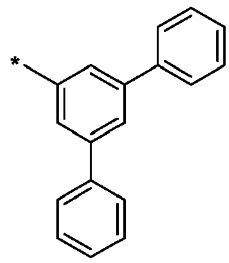 |
| (D6) | Ph | Ph | H | H | H | 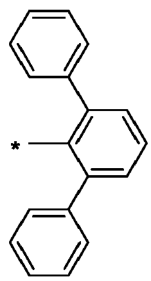 |
| (D7) | Ph | Ph | H | H | H | 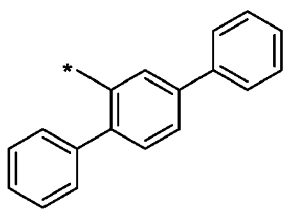 |

(continued)

| | $R^1$ | $R^2$ | $R^3$ | $R^4$ | $R^5$ | $Ar^2$ |
|---|---|---|---|---|---|---|
| (D8) | Ph | Ph | H | H | H | 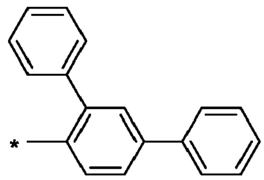 |
| (D9) | Ph | Ph | H | H | H | 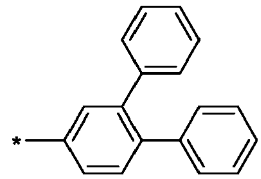 |
| (D10) | Ph | Ph | H | H | H | 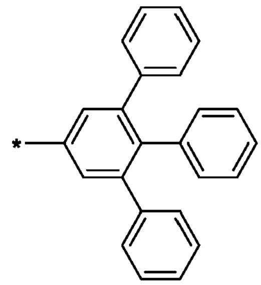 |
| (D11) | Ph | Ph | H | H | H | 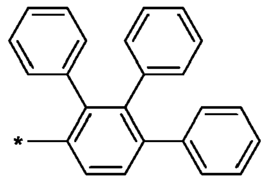 |
| (D12) | Ph | Ph | H | H | H | 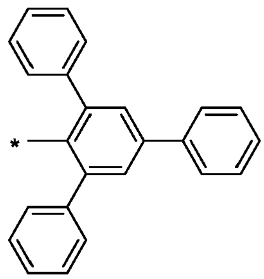 |
| (D13) | Ph | Ph | H | H | H | 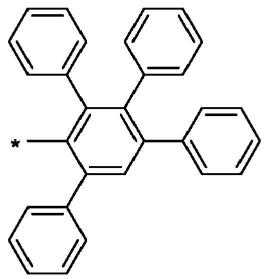 |

(continued)

| | $R^1$ | $R^2$ | $R^3$ | $R^4$ | $R^5$ | $Ar^2$ |
|---|---|---|---|---|---|---|
| (D14) | Ph | Ph | H | H | H | 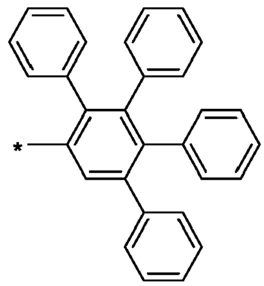 |
| (D15) | Ph | Ph | H | H | H | 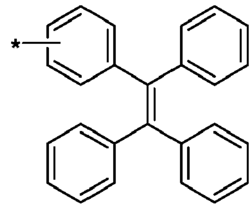 |
| (D16) | Ph | Ph | H | H | H | 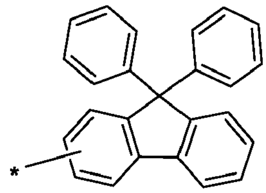 |
| (D17) | Ph | Ph | H | H | H | 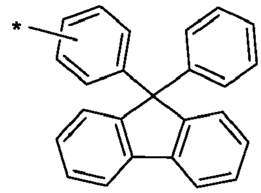 |
| (D18) | Ph | Ph | H | H | H | 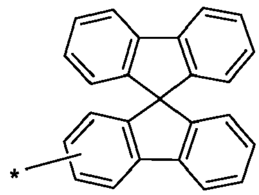 |
| (D19) | Ph | H | Ph | H | H | 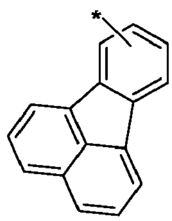 |
| (D20) | Ph | H | Ph | H | H | 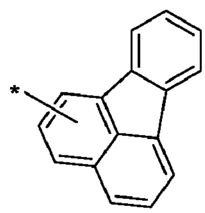 |

(continued)

| | $R^1$ | $R^2$ | $R^3$ | $R^4$ | $R^5$ | $Ar^2$ |
|---|---|---|---|---|---|---|
| (D21) | Ph | H | Ph | H | H | 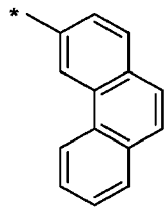 |
| (D22) | Ph | H | Ph | H | H | 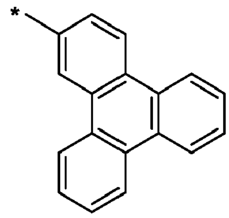 |
| (D23) | Ph | H | Ph | H | H | 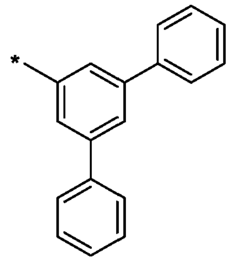 |
| (D24) | Ph | H | Ph | H | H | 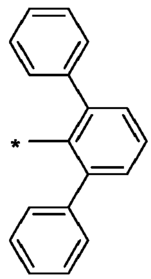 |
| (D25) | Ph | H | Ph | H | H | 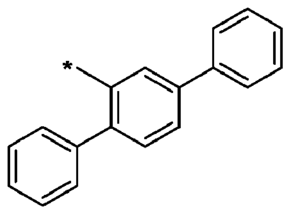 |
| (D26) | Ph | H | Ph | H | H | 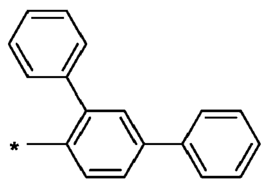 |
| (D27) | Ph | H | Ph | H | H | 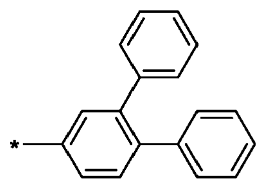 |

(continued)

| | $R^1$ | $R^2$ | $R^3$ | $R^4$ | $R^5$ | $Ar^2$ |
|---|---|---|---|---|---|---|
| (D28) | Ph | H | Ph | H | H | 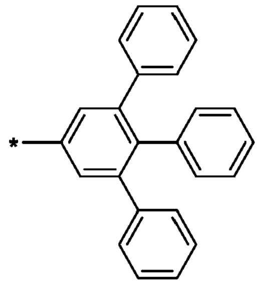 |
| (D29) | Ph | H | Ph | H | H | 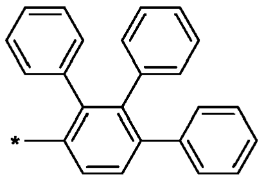 |
| (D30) | Ph | H | Ph | H | H | 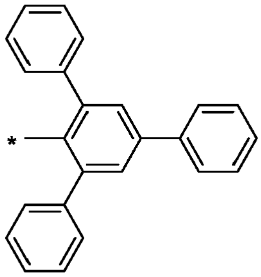 |
| (D31) | Ph | H | Ph | H | H | 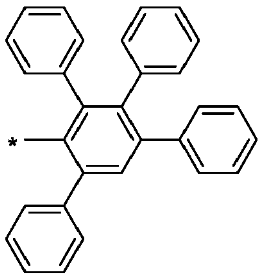 |
| (D32) | Ph | H | Ph | H | H | 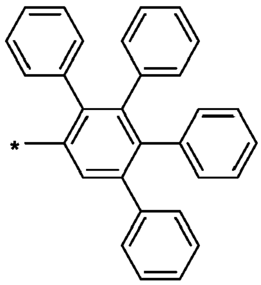 |
| (D33) | Ph | H | Ph | H | H | 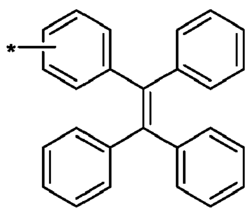 |

(continued)

| | $R^1$ | $R^2$ | $R^3$ | $R^4$ | $R^5$ | $Ar^2$ |
|---|---|---|---|---|---|---|
| (D34) | Ph | H | Ph | H | H | 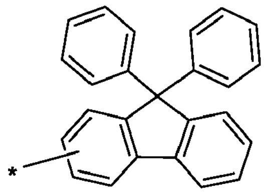 |
| (D35) | Ph | H | Ph | H | H | 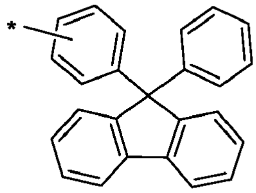 |
| (D36) | Ph | H | Ph | H | H | 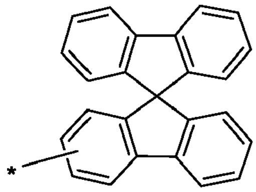 |
| (D37) | H | Ph | Ph | H | H | 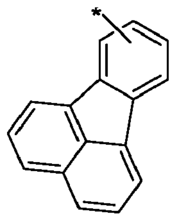 |
| (D38) | H | Ph | Ph | H | H | 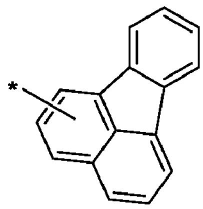 |
| (D39) | H | Ph | Ph | H | H | 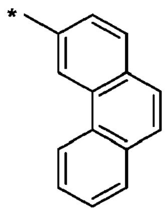 |
| (D40) | H | Ph | Ph | H | H | 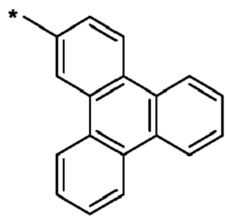 |

(continued)

| | $R^1$ | $R^2$ | $R^3$ | $R^4$ | $R^5$ | $Ar^2$ |
|---|---|---|---|---|---|---|
| (D41) | H | Ph | Ph | H | H | 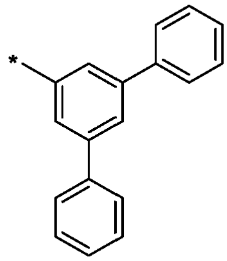 |
| (D42) | H | Ph | Ph | H | H | 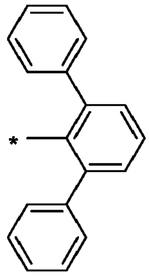 |
| (D43) | H | Ph | Ph | H | H | 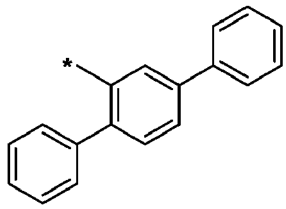 |
| (D44) | H | Ph | Ph | H | H | 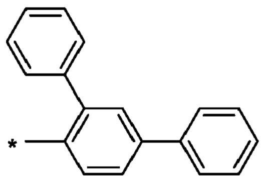 |
| (D45) | H | Ph | Ph | H | H | 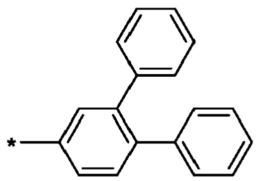 |
| (D46) | H | Ph | Ph | H | H | 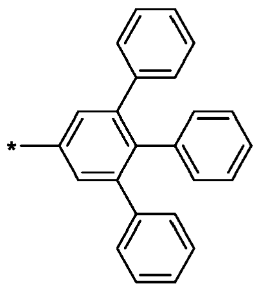 |

(continued)

| | R1 | R2 | R3 | R4 | R5 | Ar2 |
|---|---|---|---|---|---|---|
| (D47) | H | Ph | Ph | H | H | 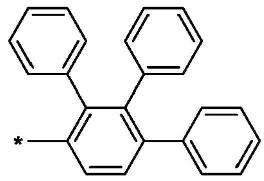 |
| (D48) | H | Ph | Ph | H | H | 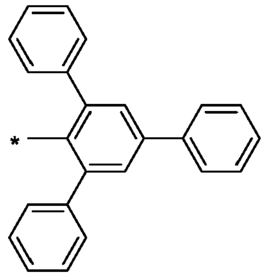 |
| (D49) | H | Ph | Ph | H | H | 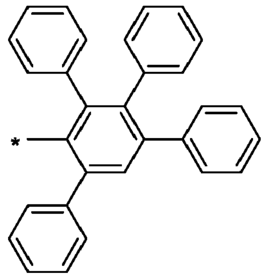 |
| (D50) | H | Ph | Ph | H | H | 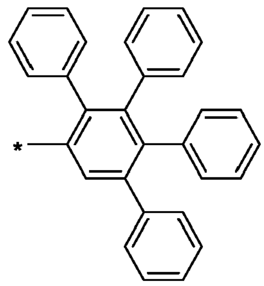 |
| (D51) | H | Ph | Ph | H | H | 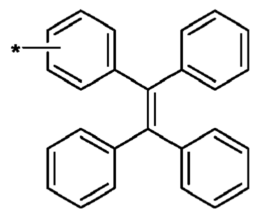 |
| (D52) | H | Ph | Ph | H | H | 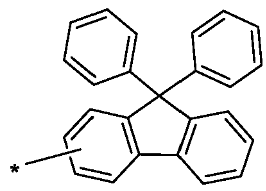 |

(continued)

| | $R^1$ | $R^2$ | $R^3$ | $R^4$ | $R^5$ | $Ar^2$ |
|---|---|---|---|---|---|---|
| (D53) | H | Ph | Ph | H | H | 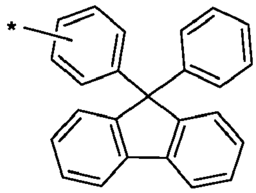 |
| (D54) | H | Ph | Ph | H | H | 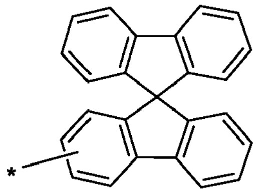 |
| (D55) | Ph | Ph | Ph | H | H | 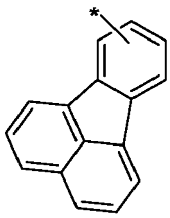 |
| (D56) | Ph | Ph | Ph | H | H | 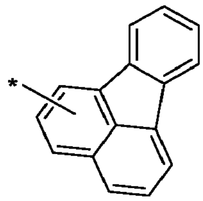 |
| (D57) | Ph | Ph | Ph | H | H | 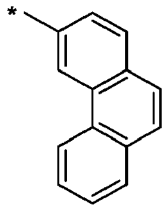 |
| (D58) | Ph | Ph | Ph | H | H | 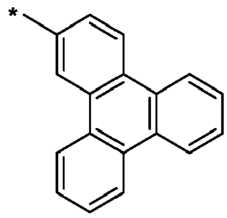 |
| (D59) | Ph | Ph | Ph | H | H | 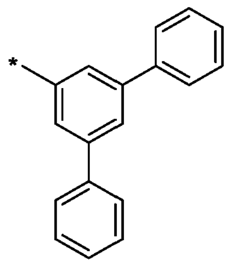 |

(continued)

| | $R^1$ | $R^2$ | $R^3$ | $R^4$ | $R^5$ | $Ar^2$ |
|---|---|---|---|---|---|---|
| (D60) | Ph | Ph | Ph | H | H | 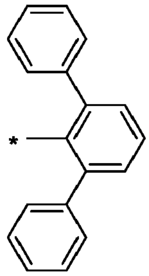 |
| (D61) | Ph | Ph | Ph | H | H | 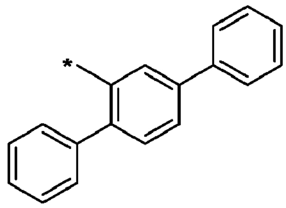 |
| (D62) | Ph | Ph | Ph | H | H | 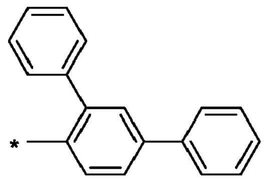 |
| (D63) | Ph | Ph | Ph | H | H | 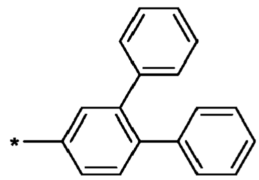 |
| (D64) | Ph | Ph | Ph | H | H | 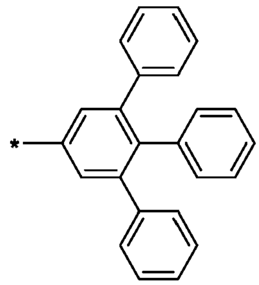 |
| (D65) | Ph | Ph | Ph | H | H | 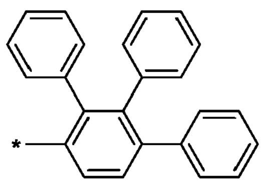 |

(continued)

| | $R^1$ | $R^2$ | $R^3$ | $R^4$ | $R^5$ | $Ar^2$ |
|---|---|---|---|---|---|---|
| (D66) | Ph | Ph | Ph | H | H | 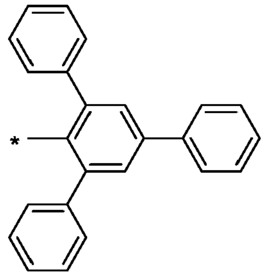 |
| (D67) | Ph | Ph | Ph | H | H | 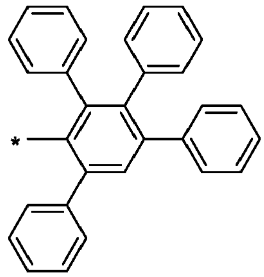 |
| (D68) | Ph | Ph | Ph | H | H | 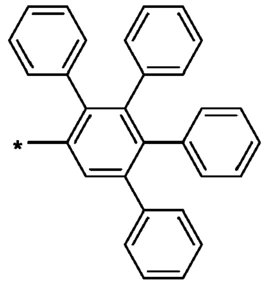 |
| (D69) | Ph | Ph | Ph | H | H | 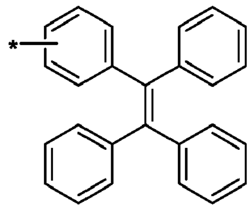 |
| (D70) | Ph | Ph | Ph | H | H | 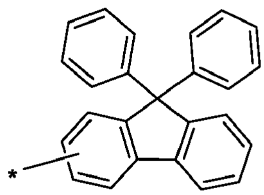 |
| (D71) | Ph | Ph | Ph | H | H | 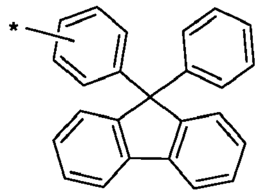 |

(continued)

| | $R^1$ | $R^2$ | $R^3$ | $R^4$ | $R^5$ | $Ar^2$ |
|---|---|---|---|---|---|---|
| (D72) | Ph | Ph | Ph | H | H | 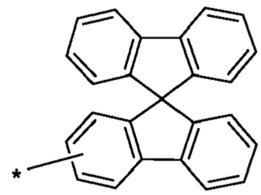 |
| (D73) | H | Ph | Ph | Ph | H | 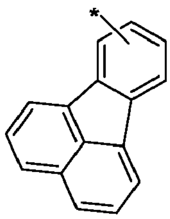 |
| (D74) | H | Ph | Ph | Ph | H | 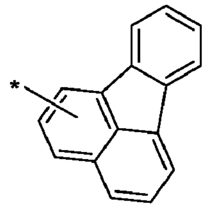 |
| (D75) | H | Ph | Ph | Ph | H | 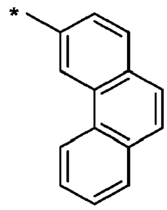 |
| (D76) | H | Ph | Ph | Ph | H | 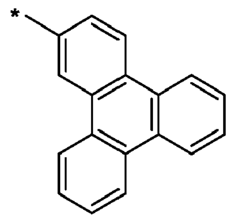 |
| (D77) | H | Ph | Ph | Ph | H | 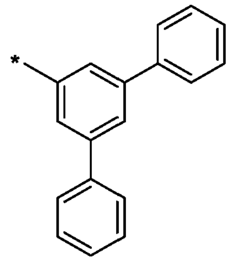 |

(continued)

| | $R^1$ | $R^2$ | $R^3$ | $R^4$ | $R^5$ | $Ar^2$ |
|---|---|---|---|---|---|---|
| (D78) | H | Ph | Ph | Ph | H | 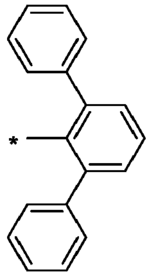 |
| (D79) | H | Ph | Ph | Ph | H | 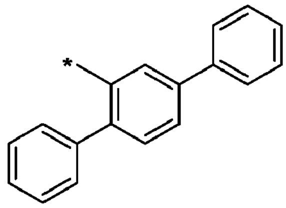 |
| (D80) | H | Ph | Ph | Ph | H | 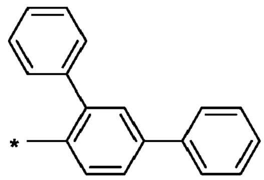 |
| (D81) | H | Ph | Ph | Ph | H | 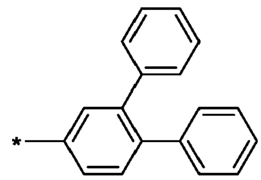 |
| (D82) | H | Ph | Ph | Ph | H | 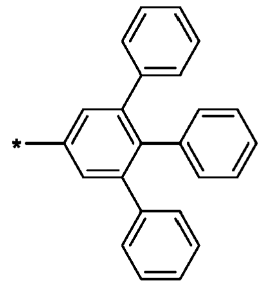 |
| (D83) | H | Ph | Ph | Ph | H | 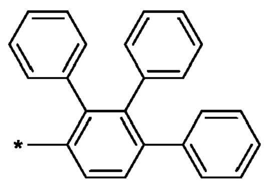 |

(continued)

| | $R^1$ | $R^2$ | $R^3$ | $R^4$ | $R^5$ | $Ar^2$ |
|---|---|---|---|---|---|---|
| (D84) | H | Ph | Ph | Ph | H | 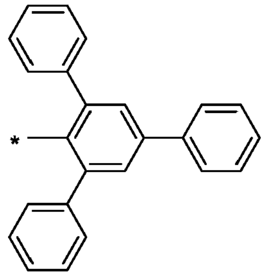 |
| (D85) | H | Ph | Ph | Ph | H | 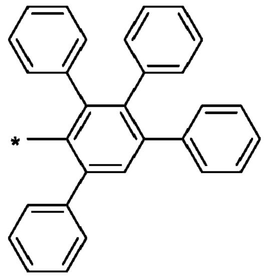 |
| (D86) | H | Ph | Ph | Ph | H | 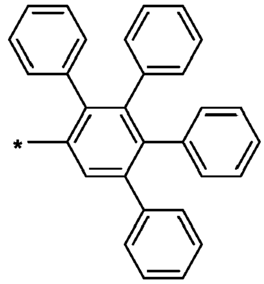 |
| (D87) | H | Ph | Ph | Ph | H | 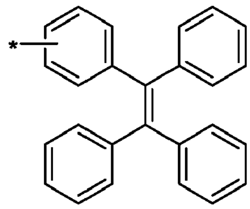 |
| (D88) | H | Ph | Ph | Ph | H | 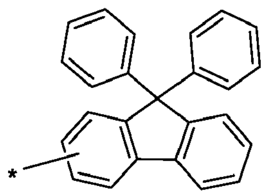 |
| (D89) | H | Ph | Ph | Ph | H | 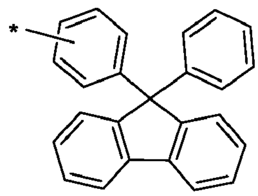 |

(continued)

| | $R^1$ | $R^2$ | $R^3$ | $R^4$ | $R^5$ | $Ar^2$ |
|---|---|---|---|---|---|---|
| (D90) | H | Ph | Ph | Ph | H | 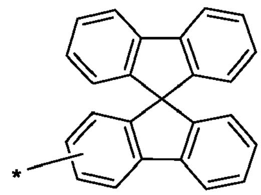 |
| (D91) | Ph | Ph | Ph | Ph | H | 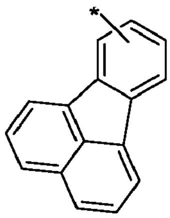 |
| (D92) | Ph | Ph | Ph | Ph | H | 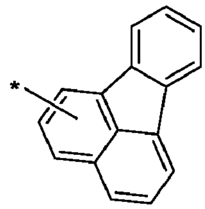 |
| (D93) | Ph | Ph | Ph | Ph | H | 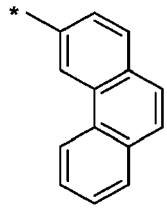 |
| (D94) | Ph | Ph | Ph | Ph | H | 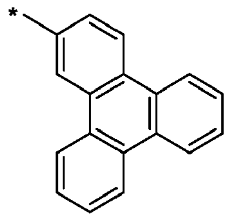 |
| (D95) | Ph | Ph | Ph | Ph | H | 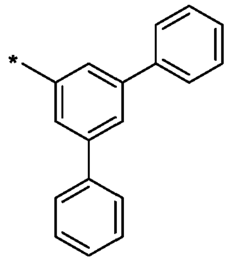 |

(continued)

| | $R^1$ | $R^2$ | $R^3$ | $R^4$ | $R^5$ | $Ar^2$ |
|---|---|---|---|---|---|---|
| (D96) | Ph | Ph | Ph | Ph | H | 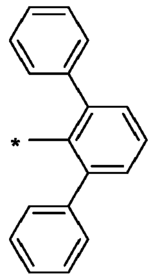 |
| (D97) | Ph | Ph | Ph | Ph | H | 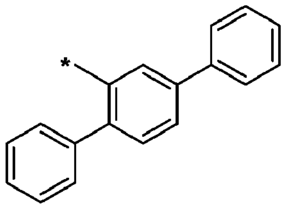 |
| (D98) | Ph | Ph | Ph | Ph | H | 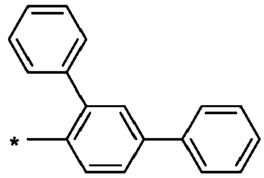 |
| (D99) | Ph | Ph | Ph | Ph | H | 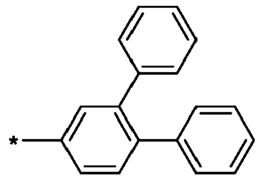 |
| (D100) | Ph | Ph | Ph | Ph | H | 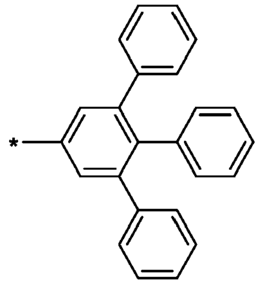 |
| (D101) | Ph | Ph | Ph | Ph | H | 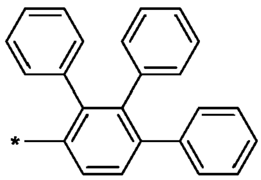 |

(continued)

| | $R^1$ | $R^2$ | $R^3$ | $R^4$ | $R^5$ | $Ar^2$ |
|---|---|---|---|---|---|---|
| (D102) | Ph | Ph | Ph | Ph | H | 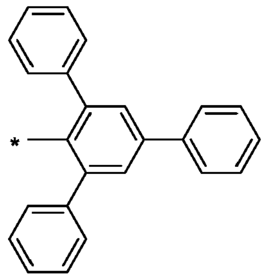 |
| (D103) | Ph | Ph | Ph | Ph | H | 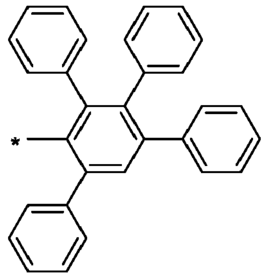 |
| (D104) | Ph | Ph | Ph | Ph | H | 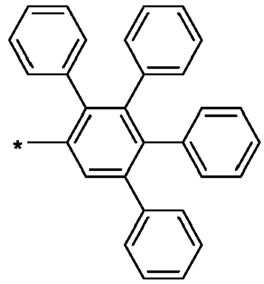 |
| (D105) | Ph | Ph | Ph | Ph | H | 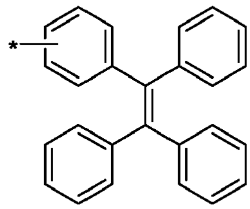 |
| (D106) | Ph | Ph | Ph | Ph | H | 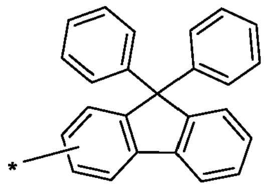 |
| (D107) | Ph | Ph | Ph | Ph | H | 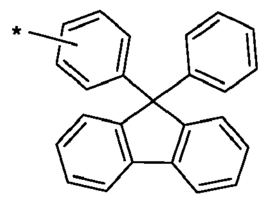 |

(continued)

| | $R^1$ | $R^2$ | $R^3$ | $R^4$ | $R^5$ | $Ar^2$ |
|---|---|---|---|---|---|---|
| (D108) | Ph | Ph | Ph | Ph | H | 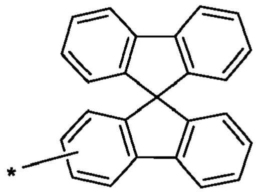 |

According to another embodiment of the present invention the compound of formula 1 can be selected from D109 to D217 as outlined in Table 2, wherein n is 1; $X^1$, $X^2$ and $X^3$ are N; $Ar^1$ is phenyl; and $R^1$, $R^2$, $R^3$, $R^4$, $R^5$ and $Ar^2$. Ph stands for phenyl.

Table 2

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D109) | Ph | Ph | H | H | H | 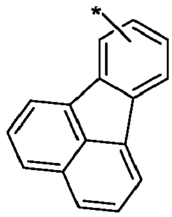 |
| (D110) | Ph | Ph | H | H | H | 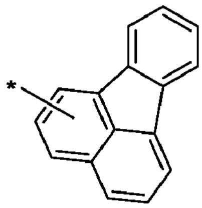 |
| (D111) | Ph | Ph | H | H | H | 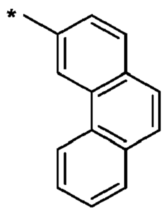 |
| (D112) | Ph | Ph | H | H | H | 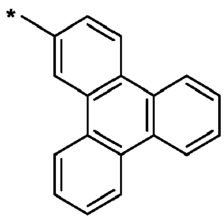 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D113) | Ph | Ph | H | H | H | 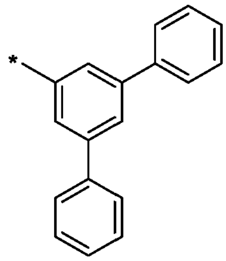 |
| (D114) | Ph | Ph | H | H | H | 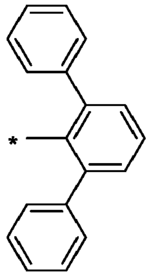 |
| (D115) | Ph | Ph | H | H | H | 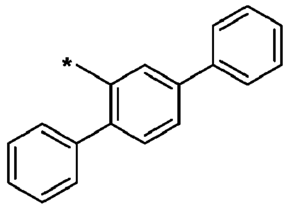 |
| (D116) | Ph | Ph | H | H | H | 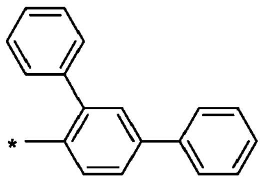 |
| (D117) | Ph | Ph | H | H | H | 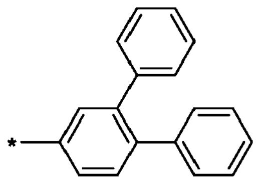 |
| (D118) | Ph | Ph | H | H | H | 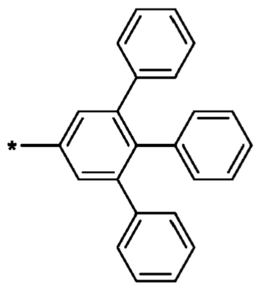 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D119) | Ph | Ph | H | H | H | 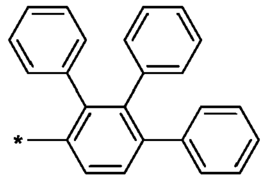 |
| (D120) | Ph | Ph | H | H | H | 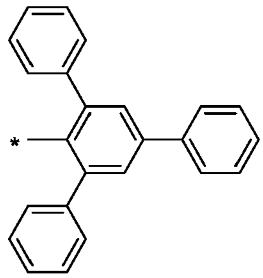 |
| (D121) | Ph | Ph | H | H | H | 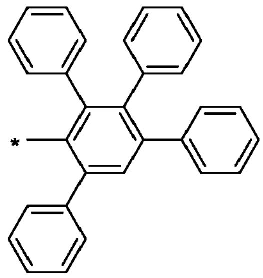 |
| (D122) | Ph | Ph | H | H | H | 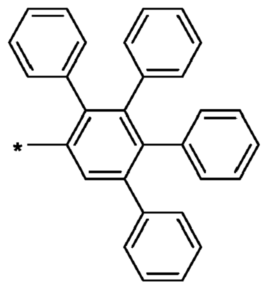 |
| (D123) | Ph | Ph | H | H | H | 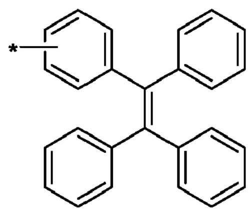 |
| (D124) | Ph | Ph | H | H | H | 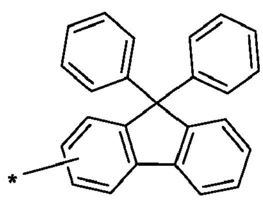 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D125) | Ph | Ph | H | H | H | 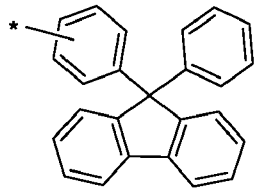 |
| (D126) | Ph | Ph | H | H | H | 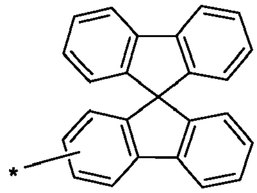 |
| (D127) | Ph | H | Ph | H | H | 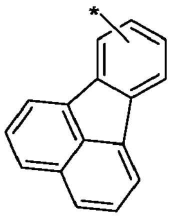 |
| (D128) | Ph | H | Ph | H | H | 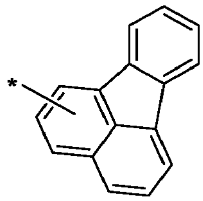 |
| (D129) | Ph | H | Ph | H | H | 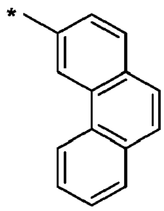 |
| (D130) | Ph | H | Ph | H | H | 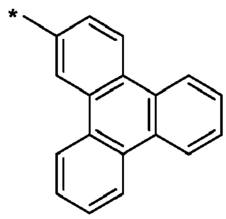 |
| (D131) | Ph | H | Ph | H | H | 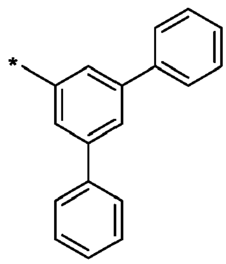 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D132) | Ph | H | Ph | H | H | 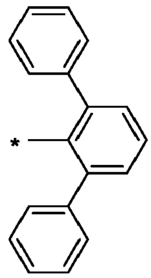 |
| (D133) | Ph | H | Ph | H | H | 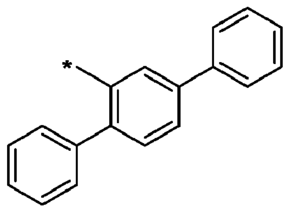 |
| (D134) | Ph | H | Ph | H | H | 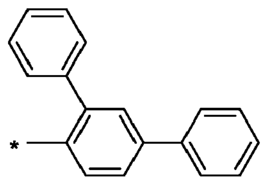 |
| (D135) | Ph | H | Ph | H | H | 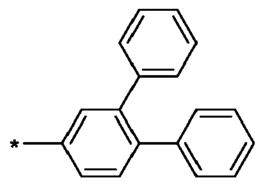 |
| (D136) | Ph | H | Ph | H | H | 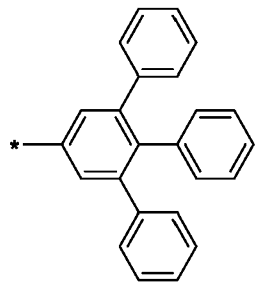 |
| (D137) | Ph | H | Ph | H | H | 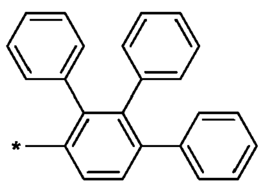 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D138) | Ph | H | Ph | H | H | 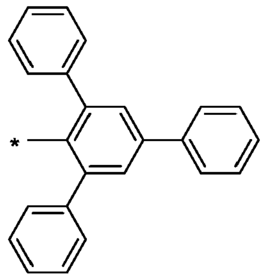 |
| (D139) | Ph | H | Ph | H | H | 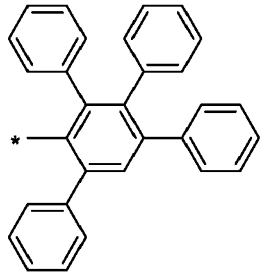 |
| (D140) | Ph | H | Ph | H | H | 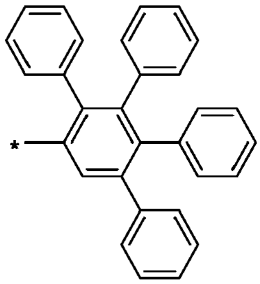 |
| (D141) | Ph | H | Ph | H | H | 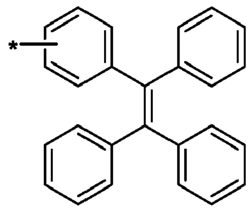 |
| (D142) | Ph | H | Ph | H | H | 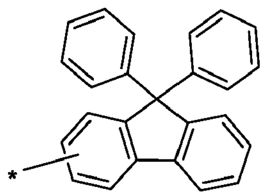 |
| (D143) | Ph | H | Ph | H | H | 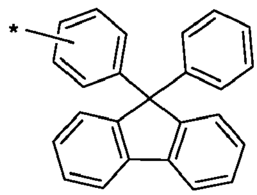 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D144) | Ph | H | Ph | H | H | 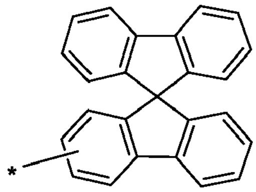 |
| (D145) | H | Ph | Ph | H | H | 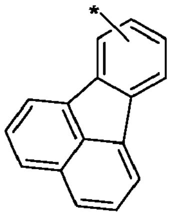 |
| (D146) | H | Ph | Ph | H | H | 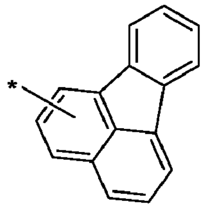 |
| (D147) | H | Ph | Ph | H | H | 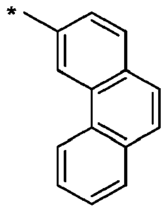 |
| (D148) | H | Ph | Ph | H | H | 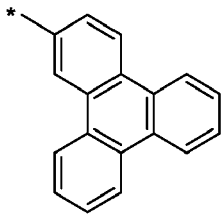 |
| (D149) | H | Ph | Ph | H | H | 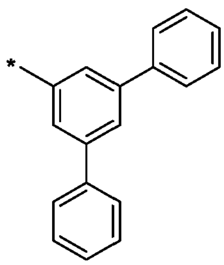 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D150) | H | Ph | Ph | H | H | 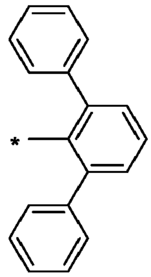 |
| (D151) | H | Ph | Ph | H | H | 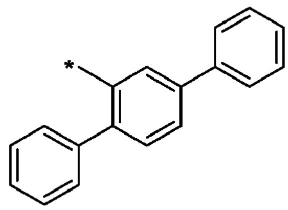 |
| (D152) | H | Ph | Ph | H | H | 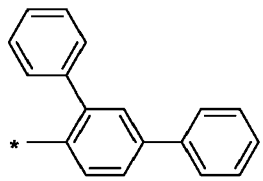 |
| (D153) | H | Ph | Ph | H | H | 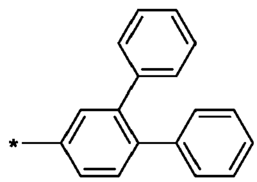 |
| (D154) | H | Ph | Ph | H | H | 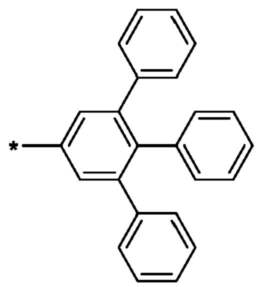 |
| (D155) | H | Ph | Ph | H | H | 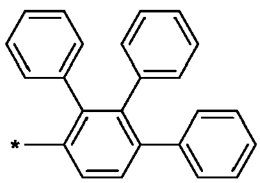 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D156) | H | Ph | Ph | H | H | 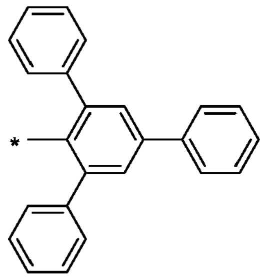 |
| (D157) | H | Ph | Ph | H | H | 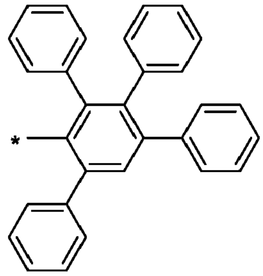 |
| (D158) | H | Ph | Ph | H | H | 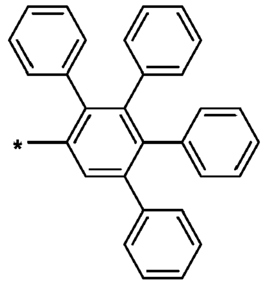 |
| (D159) | H | Ph | Ph | H | H | 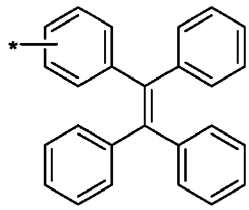 |
| (D160) | H | Ph | Ph | H | H | 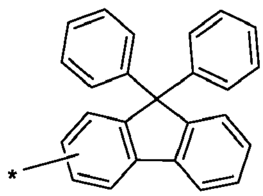 |
| (D161) | H | Ph | Ph | H | H | 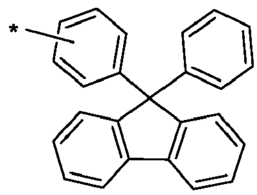 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D162) | H | Ph | Ph | H | H | 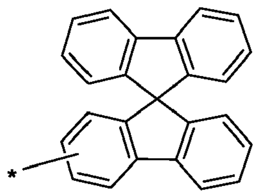 |
| (D163) | Ph | Ph | Ph | H | H | 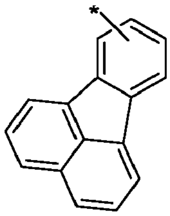 |
| (D164) | Ph | Ph | Ph | H | H | 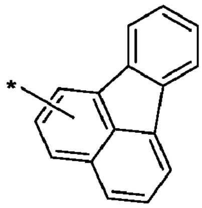 |
| (D165) | Ph | Ph | Ph | H | H | 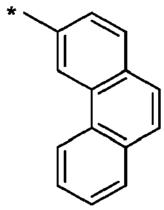 |
| (D166) | Ph | Ph | Ph | H | H | 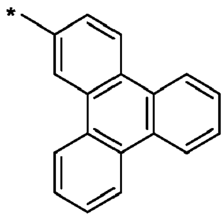 |
| (D167) | Ph | Ph | Ph | H | H | 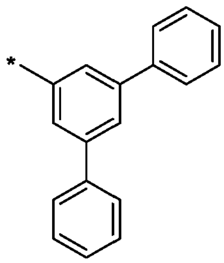 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D168) | Ph | Ph | Ph | H | H | 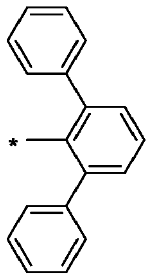 |
| (D169) | Ph | Ph | Ph | H | H | 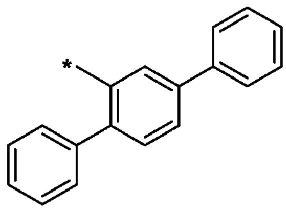 |
| (D170) | Ph | Ph | Ph | H | H | 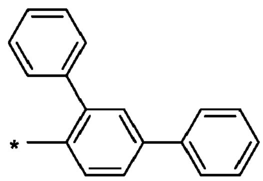 |
| (D171) | Ph | Ph | Ph | H | H | 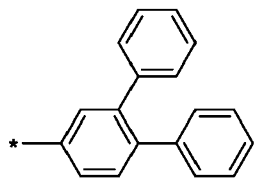 |
| (D172) | Ph | Ph | Ph | H | H | 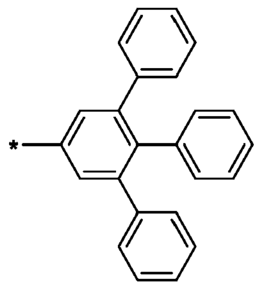 |
| (D173) | Ph | Ph | Ph | H | H | 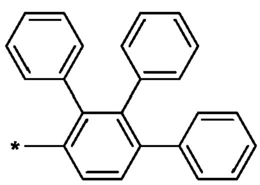 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D174) | Ph | Ph | Ph | H | H | 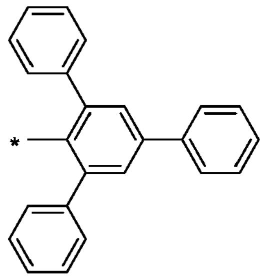 |
| (D175) | Ph | Ph | Ph | H | H | 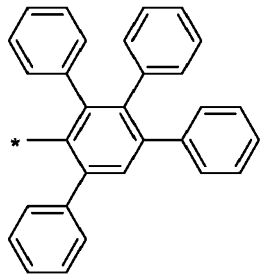 |
| (D176) | Ph | Ph | Ph | H | H | 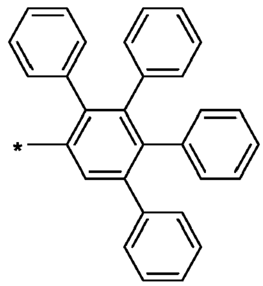 |
| (D177) | Ph | Ph | Ph | H | H | 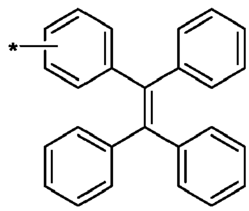 |
| (D178) | Ph | Ph | Ph | H | H | 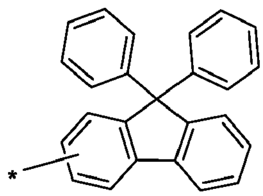 |
| (D179) | Ph | Ph | Ph | H | H | 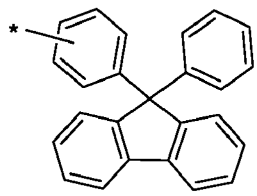 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D180) | Ph | Ph | Ph | H | H | 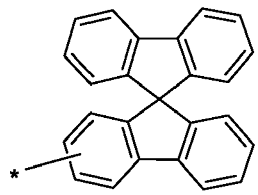 |
| (D181) | H | Ph | Ph | Ph | H | 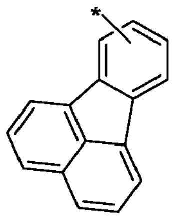 |
| (D182) | H | Ph | Ph | Ph | H | 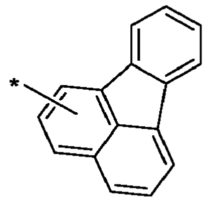 |
| (D183) | H | Ph | Ph | Ph | H | 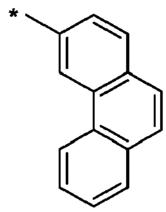 |
| (D184) | H | Ph | Ph | Ph | H | 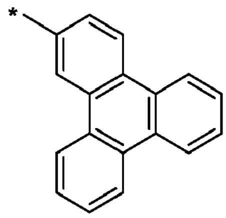 |
| (D185) | H | Ph | Ph | Ph | H | 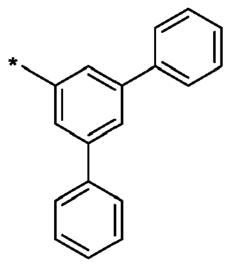 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D186) | H | Ph | Ph | Ph | H | 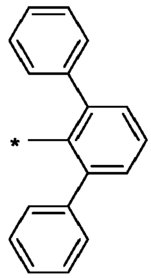 |
| (D187) | H | Ph | Ph | Ph | H | 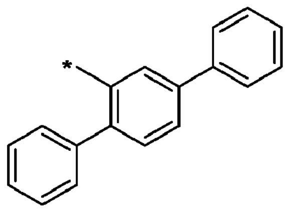 |
| (D188) | H | Ph | Ph | Ph | H | 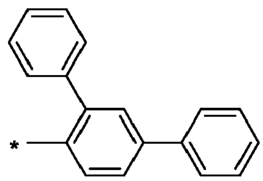 |
| (D189) | H | Ph | Ph | Ph | H | 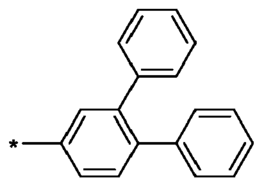 |
| (D190) | H | Ph | Ph | Ph | H | 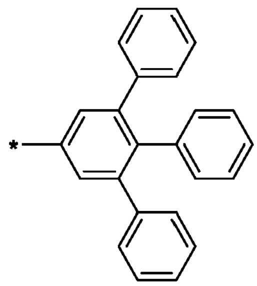 |
| (D191) | H | Ph | Ph | Ph | H | 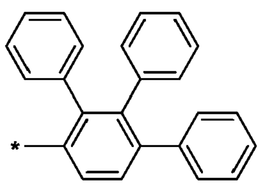 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D192) | H | Ph | Ph | Ph | H | 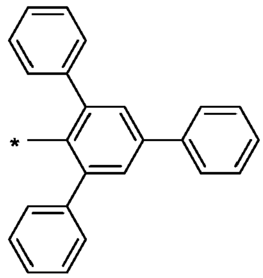 |
| (D193) | H | Ph | Ph | Ph | H | 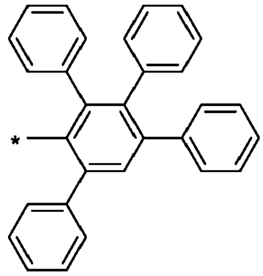 |
| (D194) | H | Ph | Ph | Ph | H | 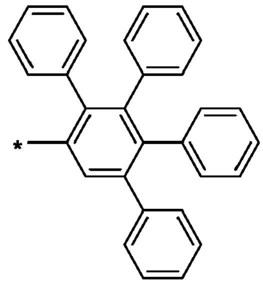 |
| (D195) | H | Ph | Ph | Ph | H | 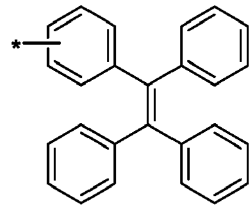 |
| (D196) | H | Ph | Ph | Ph | H | 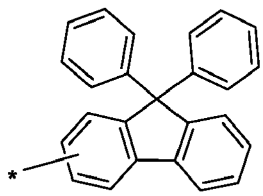 |
| (D197) | H | Ph | Ph | Ph | H | 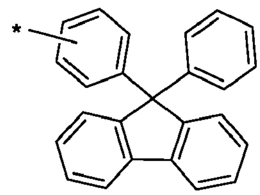 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D198) | H | Ph | Ph | Ph | H | 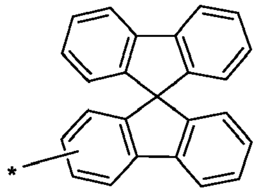 |
| (D199) | Ph | Ph | Ph | Ph | H | 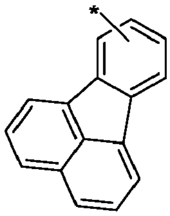 |
| (D200) | Ph | Ph | Ph | Ph | H | 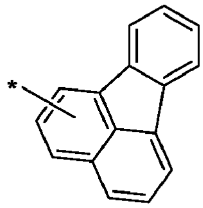 |
| (D201) | Ph | Ph | Ph | Ph | H | 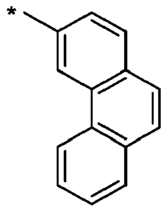 |
| (D202) | Ph | Ph | Ph | Ph | H | 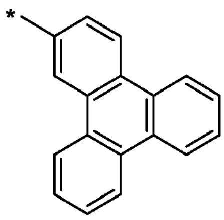 |
| (D203) | Ph | Ph | Ph | Ph | H | 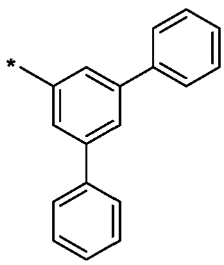 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D204) | Ph | Ph | Ph | Ph | H | 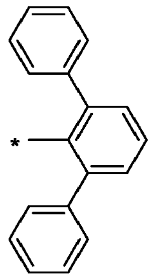 |
| (D205) | Ph | Ph | Ph | Ph | H | 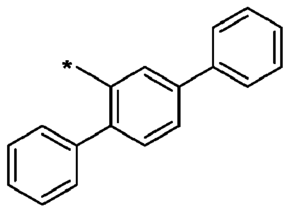 |
| (D206) | Ph | Ph | Ph | Ph | H | 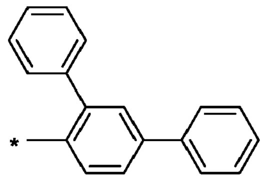 |
| (D207) | Ph | Ph | Ph | Ph | H | 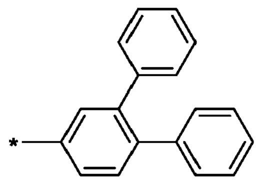 |
| (D208) | Ph | Ph | Ph | Ph | H | 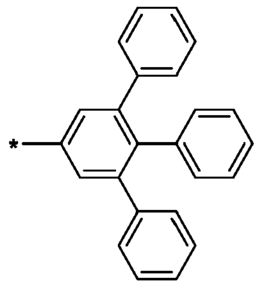 |
| (D209) | Ph | Ph | Ph | Ph | H | 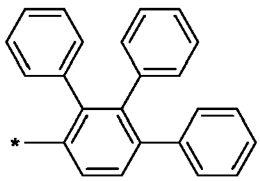 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D210) | Ph | Ph | Ph | Ph | H | 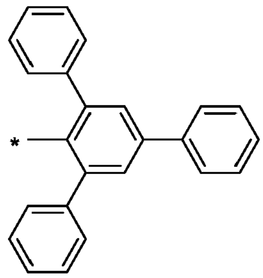 |
| (D211) | Ph | Ph | Ph | Ph | H | 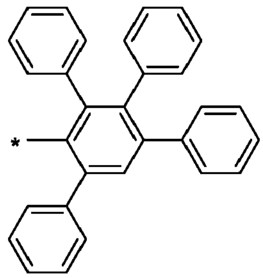 |
| (D212) | Ph | Ph | Ph | Ph | H | 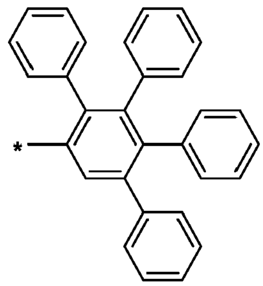 |
| (D213) | Ph | Ph | Ph | Ph | H | 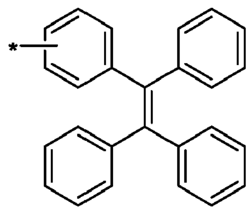 |
| (D214) | Ph | Ph | Ph | Ph | H | 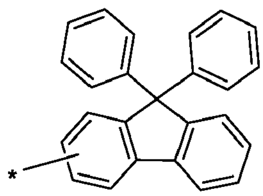 |
| (D215) | Ph | Ph | Ph | Ph | H | 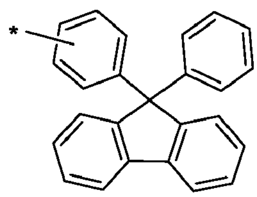 |

(continued)

| Compounds D109 to D216 of formula 1, wherein n is 1; $X^1$, $X^2$ and $X^3$ are N; $Ar^1$ is phenyl | | | | | | |
|---|---|---|---|---|---|---|
| | $R^1$ | $R^2$ | $R^3$ | $R^4$ | $R^5$ | $Ar^2$ |
| (D216) | Ph | Ph | Ph | Ph | H | 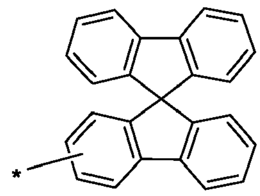 |

According to another embodiment, the compound of formula 1 is selected from F1:

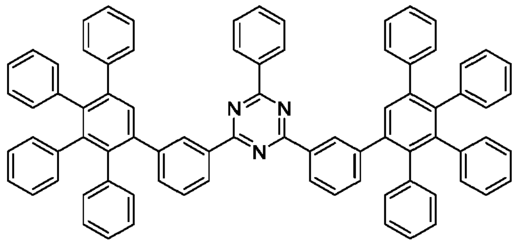

(F1).

According to an aspect the compound of formula 1 can be used as a matrix material for a dopant material.

According to an aspect the layer material can be an organic semiconductor layer, which is used for an organic electronic device. For example, the organic electronic device can be an OLED or there like.

The compounds represented by formula 1 have strong electron transport characteristics to increase charge mobility and/or stability and thereby to improve luminance efficiency, voltage characteristics, and/or lifetime characteristics.

The compounds represented by formula 1 have high electron mobility and a low operating voltage.

The compounds represented by formula 1 and an organic semiconductor layer consisting or comprising of compound of formula 1 may be non-emissive.

In the context of the present specification the term "essentially non-emissive" or "non-emitting" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconductor layer comprising the compound of formula 1 is essentially non-emissive or non-emitting.

The term "free of', "does not contain", "does not comprise" does not exclude impurities which may be present in the compounds prior to deposition. Impurities have no technical effect with respect to the object achieved by the present invention.

The operating voltage, also named U, is measured in Volt (V) at 10 milliAmpere per square be centimeter (mA/cm2).

The candela per Ampere efficiency, also named cd/A efficiency, is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The rate onset temperature is measured in °C and describes the VTE source temperature at which measurable evaporation of a compound commences at a pressure of less than $10^{-5}$ mbar.

The term "OLED", "organic light emitting diode", "organic light emitting device", "organic optoelectronic device" and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

As used herein, "weight percent", "wt.-%", "percent by weight", "% by weight", and variations thereof refer to a composition, component, substance or agent as the weight of that composition, component, substance or agent of the respective electron transport layer divided by the total weight of the composition thereof and multiplied by 100. It is understood that the total weight percent amount of all components, substances or agents of the respective electron transport layer are selected such that it does not exceed 100 wt.-%.

As used herein, "volume percent", "vol.-%", "percent by volume", "% by volume", and variations thereof refer to an elemental metal, a composition, component, substance or agent as the volume of that elemental metal, component, substance or agent of the respective electron transport layer divided by the total volume of the respective electron transport layer thereof and multiplied by 100. It is understood that the total volume percent amount of all elemental metal, components, substances or agents of the respective cathode electrode layer are selected such that it does not exceed 100 vol.-%.

All numeric values are herein assumed to be modified by the term "about", whether or not explicitly indicated. As used herein, the term "about" refers to variation in the numerical quantity that can occur.

Whether or not modified by the term "about", the claims include equivalents to the quantities.

It should be noted that, as used in this specification and the appended claims, the singular forms "a", "an", and "the" include plural referents unless the content clearly dictates otherwise.

It should be noted that, as used in this specification and the appended claims, "*" if not otherwise defined indicates the chemical bonding position.

The anode electrode and cathode electrode may be described as anode electrode / cathode electrode or anode electrode / cathode electrode or anode electrode layer / cathode electrode layer.

According to another aspect, an organic optoelectronic device comprises an anode layer and a cathode layer facing each other and at least one organic semiconductor layer between the anode layer and the cathode layer, wherein the organic semiconductor layer comprises or consists of the compound of formula 1.

According to yet another aspect, a display device comprising the organic electronic device, which can be an organic optoelectronic device, is provided.

In the present specification, when a definition is not otherwise provided, an "alkyl group" may refer to an aliphatic hydrocarbon group. The alkyl group may refer to "a saturated alkyl group" without any double bond or triple bond. The alkyl group may be a linear, cyclic or branched alkyl group.

The term "alkyl group" includes $C_1$ to $C_{16}$ alkyl, $C_3$ to $C_{16}$ branched alkyl, and $C_3$ to $C_{16}$ cyclic alkyl.

The alkyl group may be a $C_1$ to $C_{16}$ alkyl group, or preferably a $C_1$ to $C_{12}$ alkyl group. More specifically, the alkyl group may be a $C_1$ to $C_{14}$ alkyl group, or preferably a $C_1$ to $C_{10}$ alkyl group or a $C_1$ to $C_6$ alkyl group. For example, a $C_1$ to $C_4$ alkyl group comprises 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, and the like.

In the present specification"arylene group" may refer to a group comprising at least one hydrocarbon aromatic moiety, and all the elements of the hydrocarbon aromatic moiety may have p-orbitals which form conjugation, for example a phenyl group, a naphthyl group, an anthracenyl group, a phenanthrenyl group, a pyrenyl group, a fluorenyl group and the like, if not otherwise defined.

The term "heteroarylene" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S, if not otherwise defined. The heteroatom may be preferably selected from N.

A heteroarylene ring may comprise at least 1 to 3 heteroatoms. Preferably a heteroarylene ring may comprise at least 1 to 3 heteroatoms individually selected from N, S and/or O, if not otherwise defined. More preferred a heteroarylene ring may comprise at least 1 to 3 heteroatoms that can be N.

Further preferred in addition to the pyrimidine or triazine group of formula 1 at least one additional heteroaryl/ene ring may comprise at least 1 to 3 N-atoms, or at least 1 to 2-N atoms or at least one N-atom, if not otherwise defined.

According to another preferred embodiment the compound according to formula 1 may comprise:

- at least 6 to 25 aromatic rings, preferably at least 7 to 22 aromatic rings, further preferred at least 8 to 20 aromatic rings, in addition preferred at least 9 to 15 aromatic rings and more preferred at least 10 to 14 aromatic rings; wherein
- at least 1 to 5, preferably 1 to 4 or 2 to 3, are heteroaromatic rings.

According to one embodiment the compound according to formula 1:

- comprises at least about 6 to about 20 aromatic rings, preferably at least about 7 to about 18 aromatic rings, further preferred at least about 9 to about 16 aromatic rings, in addition preferred at least about 10 to about 15 aromatic rings and more preferred at least about 11 to about 14 aromatic rings; and/or
- the compound of formula 1 comprises at least about 2 to about 6, preferably about 3 to about 5 or about 2 to about 4, hetero aromatic rings, wherein the hetero atoms can be selected from N, O, S; and/or
- comprises at least one fluorene ring and at least one hetero-fluorene ring, wherein the hetero atoms can be selected from N, O, S; and/or
- comprises at least one triazine ring, or at least two triazine rings.

According to a further preferred embodiment the compound of formula 1 comprises at least 2 to 7, preferably 2 to 5, or 2 to 3 hetero aromatic rings.

According to a further preferred embodiment the compound of formula 1 comprises at least 2 to 7, preferably 2 to 5, or 2 to 3 hetero aromatic rings, wherein at least one of the aromatic rings is a five member hetero aromatic ring.

According to a further preferred embodiment the compound of formula 1 comprises at least 3 to 7, preferably 3 to 6, or 3 to 5 hetero aromatic rings, wherein at least two of the hetero aromatic rings are five member hetero-aromatic-rings.

According to one embodiment the compound according to formula 1 may comprise at least 6 to 12 non-hetero aromatic rings and 2 to 3 hetero aromatic rings.

According to one preferred embodiment the compound according to formula 1 may comprise at least 7 to 12 non-hetero aromatic rings and 2 to 5 hetero aromatic rings.

According to one preferred embodiment the compound according to formula 1 may comprise at least 7 to 11 non-hetero aromatic rings and 2 to 3 hetero aromatic rings.

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

According to another embodiment the compound of formula 1 may have a melting point of about ≥ 250° C and about ≤ 380° C, preferably about ≥ 260° C and about ≤ 370° C, further preferred about ≥ 270° C and about ≤ 360° C, in addition preferred about ≥ 280° C and about ≤ 350° C, also preferred about ≥ 290° C and about ≤ 340° C and likewise preferred about ≥ 300° C and about ≤ 330° C.

Glass transition temperature

The glass transition temperature is measured under nitrogen and using a heating rate of 10 K per min in a Mettler Toledo DSC 822e differential scanning calorimeter as described in DIN EN ISO 11357, published in March 2010. According to another embodiment the compound of formula 1 may have a glass transition temperature Tg of about ≥ 115° C and about ≤ 380° C, preferably about ≥ 150° C and about ≤ 350° C, further preferred about ≥ 155° C and about ≤ 320° C, in addition preferred about ≥ 160° C and about ≤ 200° C and also preferred about ≥ 175° C and about ≤ 190° C.

Rate onset temperature

The rate onset temperature is determined by loading 100 mg compound into a VTE source. The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset

temperature the lower is the volatility of a compound. According to another embodiment the compound of formula 1 may have a rate onset temperature $T_{RO}$ of about ≥ 200° C and about ≤ 350° C, preferably about ≥ 220° C and about ≤ 350° C, further preferred about ≥ 240° C and about ≤ 320° C, in addition preferred about ≥ 240° C and about ≤ 300° C.

## Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5. If more than one conformation is viable, the conformation with the lowest total energy is selected to determine the bond lengths of the molecules.

According to one embodiment the compounds according to formula 1 may have a dipole moment (Debye) in the range from about ≥ 0.2 to about ≤ 1.50, preferably from about ≥ 0.4 to about ≤ 1.

## Calculated HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

According to one embodiment the compounds according to formula 1 may have a LUMO energy level (eV) in the range from about - 2.00 eV to about - 1.70 eV, preferably from about - 1.9 eV to about - 1.72 eV, further preferred from about - 1.9 eV to about - 1.75 eV.

## Molar mass

The molar mass of a compound in g/mol is given by the sum of the standard atomic weight (namely, the standard relative atomic mass) of the atoms which form the compound multiplied by the molar mass constant, $M_u$. $M_u$ is $1 \times 10^{-3}$ kg/mol = 1 g/mol.According to one embodiment the compounds according to formula 1 may have a molar mass (g/mol) in the range from about 650 to about 1800 g/mol, preferably from about 680 to about 1600 g/mol, further preferred from about 700 to about 1400 g/mol.

## Technical effect

Surprisingly, it was found that the compounds of formula 1 and the inventive organic electronic devices solve the problem underlying the present invention by being superior over the organic electroluminescent devices and compounds known in the art, in particular with respect to cd/A efficiency, also referred to as current efficiency and to lifetime. At the same time the operating voltage is kept at a similar or even improved level which is important for reducing power consumption and increasing battery life, for example of a mobile display device. High cd/A efficiency is important for high efficiency and thereby increased battery life of a mobile device, for example a mobile display device. Long lifetime at high current density is important for the longevity of a device which is run at high brightness.

Additionally, the glass transition temperature is improved over compounds known in the art. A high glass transition temperature may offer a technical benefit in high temperature applications, as the morphology of the organic semiconductor layer is less likely to deteriorate. Suprisingly, it was found that the rate onset temperature is still within a range suitable for mass production, even though the molar mass of compounds of formula 1 is higher than for comparative examples.

The inventors have surprisingly found that particular good performance can be achieved when using the organic electroluminescent device as a fluorescent blue device.
The specific arrangements mentioned herein as preferred were found to be particularly advantageous.
Likewise, some compounds falling within the scope of the broadest definition of the present invention have surprisingly be found to be particularly well performing with respect to the mentioned property of cd/A efficiency and/or lifetime. These compounds are discussed herein to be particularly preferred.
Further an organic optoelectronic device having high efficiency and/or long lifetime may be realized.

Anode

A material for the anode may be a metal or a metal oxide, or an organic material, preferably a material with work function above about 4.8 eV, more preferably above about 5.1 eV, most preferably above about 5.3 eV. Preferred metals are noble metals like Pt, Au or Ag, preferred metal oxides are transparent metal oxides like ITO or IZO which may be advantageously used in bottom-emitting OLEDs having a reflective cathode.
In devices comprising a transparent metal oxide anode or a reflective metal anode, the anode may have a thickness from about 50 nm to about 100 nm, whereas semitransparent metal anodes may be as thin as from about 5 nm to about 15 nm, and non-transparent metal anodes may have a thickness from about 15 nm to about 150nm.

Hole injection layer (HIL)

The hole injection layer may improve interface properties between the anode and an organic material used for the hole transport layer, and is applied on a non-planarized anode and thus may planarize the surface of the anode. For example, the hole injection layer may include a material having a median value of the energy level of its highest occupied molecular orbital (HOMO) between the work function of the anode material and the energy level of the HOMO of the hole transport layer, in order to adjust a difference between the work function of the anode and the energy level of the HOMO of the hole transport layer.
When the hole transport region comprises a hole injection layer 36, the hole injection layer may be formed on the anode by any of a variety of methods, for example, vacuum deposition, spin coating, casting, Langmuir-Blodgett (LB) method, or the like.
When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of about 100 °C to about 500 °C, a pressure of about $10^{-6}$ Pa to about $10^{-1}$ Pa, and a deposition rate of about 0.1 to about 10 nm/sec, but the deposition conditions are not limited thereto.
When the hole injection layer is formed using spin coating, the coating conditions may vary depending on the material that is used to form the hole injection layer, and the desired structure and thermal properties of the hole injection layer to be formed. For example, the coating rate may be in the range of about 2000 rpm to about 5000 rpm, and a temperature at which heat treatment is performed to remove a solvent after coating may be in a range of about 80 °C to about 200 °C, but the coating conditions are not limited thereto.
The hole injection layer may further comprise a p-dopant to improve conductivity and/or hole injection from the anode.

p-dopant

In another aspect, the p-dopant may be homogeneously dispersed in the hole injection layer.
In another aspect, the p-dopant may be present in the hole injection layer in a higher concentration closer to the anode and in a lower concentration closer to the cathode.
The p-dopant may be one of a quinone derivative or a radialene compound but not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), 4,4',4"-((1E,1'E,1"E)-cyclopropane-1,2,3-triylidenetris(cyanomethanyl ylidene))-tris(2,3,5,6-tetrafluorobenzonitrile).
Acccording to another embodiment, the device comprising comprising a compound of formula 1 may further comprise a layer comprising a radialene compound and/or a quinodimethane compound.
In another embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have formula (XX) and/or the quinodimethane compound may have formula (XXIa) or (XXIb):

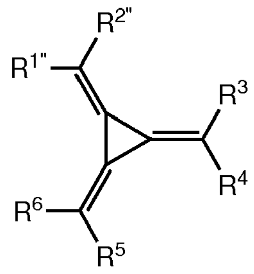
(XX)

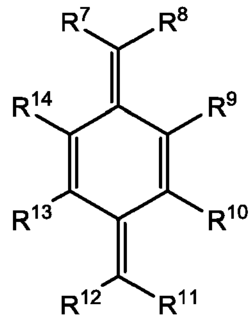
(XXIa)

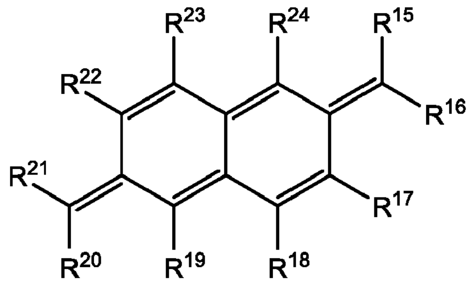
(XXIb),

wherein $R^{1''}$, $R^{2''}$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from an electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and electron withdrawing groups. Electron withdrawing group that can be suitable used are above mentioned.

Hole transport layer (HTL)

Conditions for forming the hole transport layer and the electron blocking layer may be defined based on the above-described formation conditions for the hole injection layer.

A thickness of the hole transport part of the charge transport region may be from about 10 nm to about 1000 nm, for example, about 10 nm to about 100 nm. When the hole transport part of the charge transport region comprises the hole injection layer and the hole transport layer, a thickness of the hole injection layer may be from about 10 nm to about 1000 nm, for example about 10 nm to about 100 nm and a thickness of the hole transport layer may be from about 5 nm to about 200 nm, for example about 10 nm to about 150 nm. When the thicknesses of the hole transport part of the charge transport region, the HIL, and the HTL are within these ranges, satisfactory hole transport characteristics may be obtained without a substantial increase in operating voltage.

Hole transport matrix materials used in the hole transport region are not particularly limited. Preferred are covalent compounds comprising a conjugated system of at least 6 delocalized electrons, preferably organic compounds comprising at least one aromatic ring, more preferably organic compounds comprising at least two aromatic rings, even more preferably organic compounds comprising at least three aromatic rings, most preferably organic compounds comprising at least four aromatic rings. Typical examples of hole transport matrix materials which are widely used in hole transport layers are polycyclic aromatic hydrocarbons, triarylene amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple

as reference) may be in the range 0.0 - 1.0 V, more preferably in the range 0.2 - 0.7 V, even more preferably in the range 0.3 - 0.5 V.

Buffer layer

The hole transport part of the charge transport region may further include a buffer layer.

Buffer layer that can be suitable used are disclosed in US 6 140 763, US 6 614 176 and in US2016/248022.

The buffer layer may compensate for an optical resonance distance of light according to a wavelength of the light emitted from the EML, and thus may increase efficiency.

Emission layer (EML)

The emission layer may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

A thickness of the emission layer may be about 100Å to about 1000Å, for example about 200Å to about 600Å. When the thickness of the emission layer is within these ranges, the emission layer may have improved emission characteristics without a substantial increase in operating voltage.

Emitter host

According to another embodiment, the emission layer comprises compound of formula 1 as emitter host.

The emitter host compound has at least three aromatic rings, which are independently selected from carbocyclic rings and heterocyclic rings.

Other compounds that can be used as the emitter host is an anthracene matrix compound represented by formula 400 below:

Formula 400

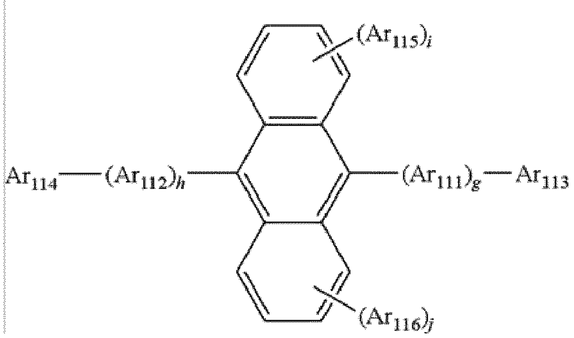

In formula 400, $Ar_{111}$ and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ arylene group; and g, h, i, and j may be each independently an integer from 0 to 4.

In some embodiments, $Ar_{111}$ and $Ar_{112}$ in formula 400 may be each independently one of a phenylene group, a naphthalene group, a phenanthrenylene group, or a pyrenylene group; or a phenylene group, a naphthalene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2.

In formula 400, $Ar_{113}$ to $Ar_{116}$ may be each independently one of

- a $C_1$-$C_{10}$ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof, a phosphoric acid group or a salt thereof,

- a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or
- a fluorenyl group

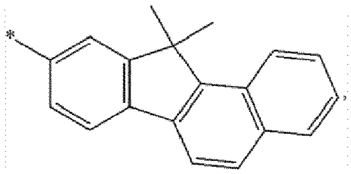;

or
- formulas 7 or 8

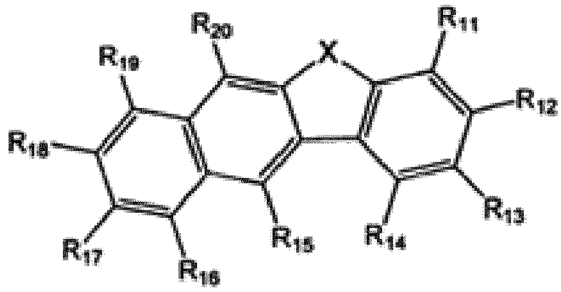

(7),

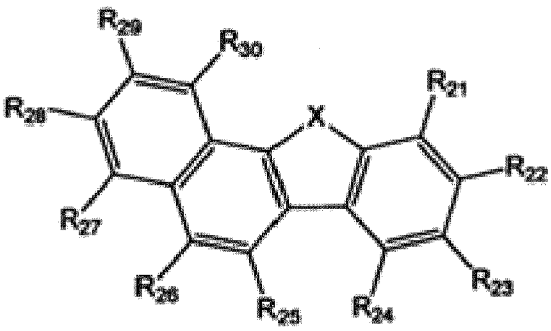

(8).

Wherein in the formulas 7 and 8, X is selected form an oxygen atom and a sulfur atom, but embodiments of the invention are not limited thereto.

In the formula 7, any one of $R_{11}$ to $R_{14}$ is used for bonding to $Ar_{111}$. $R_{11}$ to $R_{14}$ that are not used for bonding to $Ar_{111}$ and $R_{15}$ to $R_{20}$ are the same as $R_1$ to $R_8$.

In the formula 8, any one of $R_{21}$ to $R_{24}$ is used for bonding to $Ar_{111}$. $R_{21}$ to $R_{24}$ that are not used for bonding to $Ar_{111}$ and $R_{25}$ to $R_{30}$ are the same as $R_1$ to $R_8$.

Preferably, the EML host comprises between one and three heteroatoms selected from the group consisting of N, O or S. More preferred the EML host comprises one heteroatom selected from S or O.

Emitter dopant

The dopant is mixed in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The emitter may be a red, green, or blue emitter.

The dopant may be a fluorescent dopant, for example ter-fluorene, the structures are shown below. 4.4'-bis(4-diphenyl amiostyryl)biphenyl (DPAVBI, 2,5,8,11-tetra-tert-butyl perylene (TBPe), and Compound 8 below are examples of fluorescent blue dopants.

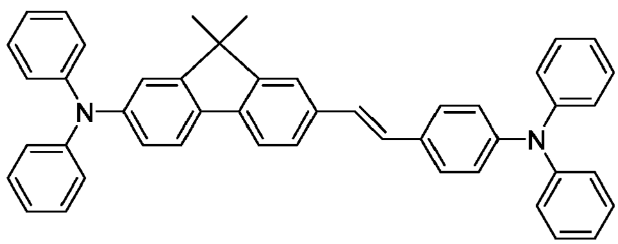

**Compound 8**

The dopant may be a phosphorescent dopant, and examples of the phosphorescent dopant may be an organic metal compound comprising Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto:

$$J_2MX \qquad (Z).$$

In formula Z, M is a metal, and J and X are the same or different, and are a ligand to form a complex compound with M.
The M may be, for example Ir, Pt, Os, Ti, Zr, Hf, Eu, Tb, Tm, Fe, Co, Ni, Ru, Rh, Pd or a combination thereof, and the J and X may be, for example a bidendate ligand.
One or more emission layers may be arranged between the anode and the cathode. To increase overall performance, two or more emission layers may be present.

Charge generation layer

A charge generation layer (also named CGL) may be arranged between the first and the second emission layer, and second and third emission layer, if present. Typically, the CGL comprises a n-type charge generation layer (also named n-CGL or electron generation layer) and a p-type charge generation layer (also named p-CGL or hole generation layer). An interlayer may be arranged between the n-type CGL and the p-type CGL.
In one aspect, the n-type CGL may comprise a compound of formula 1. The n-type CGL further comprises a metal, metal salt or organic metal complex, preferably a metal. The metal may be selected from an alkali, alkaline earth or rare earth metal.
The p-type CGL may comprise a dipyrazino[2,3-f:2',3'-h]quinoxaline, a quinone compound or a radialene compound, preferably dipyrazino[2,3-f:2',3'-h]quinoxaline-2,3,6,7,10,11-hexacarbonitrile or a compound or formula (XX) and/or a compound of formula (XXIa) or (XXIb).
In another aspect, the n-type and p-type CGL are in direct contact.

Electron transport layer (ETL)

According to another embodiment, the organic semiconductor layer that comprises compound of formula 1 is an electron transport layer. In another embodiment the electron transport layer may consist of compound of formula 1.
For example, an organic light emitting diode according to an embodiment of the present invention comprises at least one electron transport layer, and in this case, the electron transport layer comprises a compound of formula 1, or preferably of at least one compound of formulae D1 to D108 of Table 1 and D109 to D216, preferably F1.
In another embodiment, the organic electronic device comprises an electron transport region of a stack of organic layers formed by two or more electron transport layers, wherein at least one electron transport layer comprises a compound of formula 1.
The electron transport layer may include one or two or more different electron transport compounds.
According to another embodiment, a first electron transport layer comprises at least one compound of formula 1 according to the invention and a second electron transport layer comprises a matrix compound, which is selected different to the compound of formula 1 according to the invention, and may be selected from:

- an anthracene based compound or a hetero substituted anthracene based compound, preferably 2-(4-(9,10-di(naphthalen-2-yl)anthracene-2-yl)phenyl)-1-phenyl-1H-benzo[d]imidazole and/or N4,N4"-di(naphthalen-1-yl)-N4,N4"-diphenyl-[1,1':4',1"-terphenyl]-4,4"-diamine and/or
- a triazine based compound, preferably the triazine based compound comprising aryl and/or heteroaryl substituents, preferably aryl substituents, and/or
- a phosphine oxide based compound, preferably (3-(dibenzo[c,h]acridin-7-yl)phenyl)diphenylphosphine oxide and/or phenyl bis(3-(pyren-1-yl)phenyl)phosphine oxide and/or 3-Phenyl-3H-benzo[b]dinaphtho[2,1-d:1',2'-f]phosphepine-3-oxide; or
- a substituted phenanthroline compound, preferably 2,4,7,9-tetraphenyl-1,10-phenanthroline or 2,9-di(biphenyl-4-yl)-4,7-diphenyl-1,10-phenanthroline.

According to another embodiment a first electron transport layer comprises at least one compound of formula 1 according to the invention and a second electron transport layer comprises a matrix compound, which is selected different to the compound of formula 1 according to the invention, and may be selected from a phosphine oxide based compound, preferably (3-(dibenzo[c,h]acridin-7-yl)phenyl)diphenylphosphine oxide and/or phenyl bis(3-(pyren-1-yl)phenyl)phosphine oxide and/or 3-Phenyl-3H-benzo[b]dinaphtho[2,1-d:1',2'-f]phosphepine-3-oxide.

The first electron transport layer may also be described as auxiliary electron transport layer.

According to another embodiment, a first and a second electron transport layers comprise compound of formula 1, wherein the compound of formula 1 is not selected the same.

The thickness of the electron transport layer may be from about 0.5 nm to about 100 nm, for example about 2 nm to about 40 nm, preferably 2 to 10 nm. The thickness of the first electron transport layer may be from about 0.5 nm to about 100 nm, for example about 2 nm to about 40 nm, preferably 2 to 10 nm.

A thickness of an optional second electron transport layer may be about 1 nm to about 100 nm, for example about 2 nm to about 50 nm, preferably 10 to 40 nm. When the thickness of the electron transport layer is within these ranges, the electron transport layer may have satisfactory electron transporting ability without a substantial increase in operating voltage.

The electron transport layer may further comprise a monovalent or divalent metal halide or an organic monovalent or divalent metal complex, preferably an alkali halide and/or alkali organic complex.

According to another embodiment, the first and second electron transport layers comprise compound of formula 1, wherein the second electron transport layer further comprises an alkali halide and/or alkali organic complex.

Alkali halide

Alkali halides, also known as alkali metal halides, are the family of inorganic compounds with the chemical formula MX, where M is an alkali metal and X is a halogen.

M can be selected from Li, Na, Potassium, Rubidium and Cesium.

X can be selected from F, Cl, Br and J.

According to various embodiments of the present invention a lithium halide may be preferred. The lithium halide can be selected from the group comprising LiF, LiCl, LiBr and LiJ. However, most preferred is LiF.

The alkali halide is essentially non-emissive or non-emissive.

Alkali organic complex

The alkali organic complex comprises an alkali metal and at least one organic ligand. The alkali metal is preferably selected from lithium.

According to various embodiments of the present invention the organic ligand of the lithium organic complex is a quinolate, a borate, a phenolate, a pyridinolate or a Schiff base ligand;

- preferably the lithium quinolate complex has the formula III, IV or V:

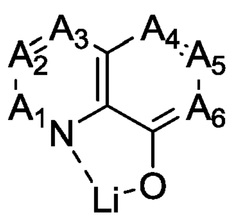 (III), 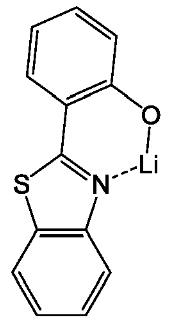 (IV), 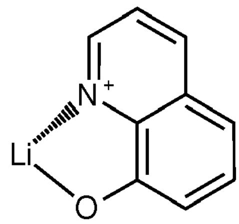 (V),

wherein

$A_1$ to $A_6$ are same or independently selected from CH, CR, N and O;
R is same or independently selected from hydrogen, halogen, alkyl or arylene or heteroarylene with 1 to 20 carbon atoms; and more preferred A1 to A6 are CH;

- preferably the borate based organic ligand is a tetra(1H-pyrazol-1-yl)borate;
- preferably the phenolate is a 2-(pyridin-2-yl)phenolate, a 2-(diphenylphosphoryl)phenolate, an imidazol phenolates, or 2-(pyridin-2-yl)phenolate and more preferred 2-(1-phenyl-1H-benzo[d]imidazol-2-yl)phenolate;
- preferably the pyridinolate is a 2-(diphenylphosphoryl)pyridin-3-olate.

According to various embodiments of the present invention the organic ligand of the alkali organic complex,

preferably of a lithium organic complex, can be a quinolate. Quinolates that can be suitable used are disclosed in WO 2013079217 A1 and incorporated by reference.

According to various embodiments of the present invention the organic ligand of the lithium organic complex can be a borate based organic ligand, Preferably the lithium organic complex is a lithium tetra(1H-pyrazol-1-yl)borate. Borate based organic ligands that can be suitable used are disclosed in WO 2013079676 A1 and incorporated by reference.

According to various embodiments of the present invention the organic ligand of the lithium organic complex can be a phenolate ligand, Preferably the lithium organic complex is a lithium 2-(diphenylphosphoryl)phenolate. Phenolate ligands that can be suitable used are disclosed in WO 2013079678 A1 and incorporated by reference.

Further, phenolate ligands can be selected from the group of pyridinolate, preferably 2-(diphenylphosphoryl)pyridin-3-olate. Pyridine phenolate ligands that can be suitable used are disclosed in JP 2008195623 and incorporated by reference.

In addition, phenolate ligands can be selected from the group of imidazol phenolates, preferably 2-(1-phenyl-1H-benzo[d]imidazol-2-yl)phenolate. Imidazol phenolate ligands that can be suitable used are disclosed in JP 2001291593 and incorporated by reference.

Also, phenolate ligands can be selected from the group of oxazol phenolates, preferably 2-(benzo[d]oxazol-2-yl)phenolate. Oxazol phenolate ligands that can be suitable used are disclosed in US 20030165711 and incorporated by reference.

The alkali organic complex may be essentially non-emissive.

## Electron injection layer (EIL)

According to another aspect of the invention, the organic electroluminescent device may further comprise an electron injection layer between the electron transport layer (first-ETL) and the cathode.
The electron injection layer (EIL) may facilitate injection of electrons from the cathode.

According to another aspect of the invention, the electron injection layer comprises:

(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or

(ii) an alkali metal complex and/or alkali metal salt, preferably the Li complex and/or salt, more preferably a Li quinolinolate, even more preferably a lithium 8-hydroxyquinolinolate, most preferably the alkali metal salt and/or complex of the second electron transport layer (second-ETL) is identical with the alkali metal salt and/or complex of the injection layer.

The electron injection layer may include at least one selected from LiF, NaCl, CsF, $Li_2O$, and BaO.

A thickness of the EIL may be from about 0.1 nm to about 10 nm, or about 0.3 nm to about 9 nm. When the thickness of the electron injection layer is within these ranges, the electron injection layer may have satisfactory electron injection ability without a substantial increase in operating voltage.

The electron injection layer may comprise a compound of formula 1.

## Cathode

A material for the cathode may be a metal, an alloy, or an electrically conductive compound that have a low work function, or a combination thereof. Specific examples of the material for the cathode may be lithium (Li), magnesium (Mg), aluminum (Al), aluminum-lithium (Al-Li), calcium (Ca), magnesium-indium (Mg-In), magnesium-silver (Mg-Ag), silver (Ag) etc. In order to manufacture a top-emission light-emitting device having a reflective anode deposited on a substrate, the cathode may be formed as a light-transmissive electrode from, for example, indium tin oxide (ITO), indium zinc oxide (IZO) or silver (Ag).

In devices comprising a transparent metal oxide cathode or a reflective metal cathode, the cathode may have a thickness from about 50 nm to about 100 nm, whereas semitransparent metal cathodes may be as thin as from about 5 nm to about 15 nm.

## Substrate

A substrate may be further disposed under the anode or on the cathode. The substrate may be a substrate that is used in a general organic light emitting diode and may be a glass substrate or a transparent plastic substrate with strong mechanical strength, thermal stability, transparency, surface smoothness, ease of handling, and water resistance.

Hereinafter, the embodiments are illustrated in more detail with reference to examples. However, the present disclosure is not limited to the following examples.

**Description of the Drawings**

These and/or other aspects and advantages of the present invention will become apparent and more readily appreciated from the following description of the exemplary embodiments, taken in conjunction with the accompanying drawings, of which:

- FIG. 1 is a schematic sectional view of an organic light-emitting diode (OLED), according to an exemplary embodiment of the present invention with an emission layer, one electron transport layer and an electron injection layer;
- FIG. 2 is a schematic sectional view of an organic light-emitting diode (OLED), according to an exemplary embodiment of the present invention with an emission layer and two electron transport layers;
- FIG. 3 is a schematic sectional view of an OLED, according to an exemplary embodiment of the present invention with an emission layer and three electron transport layers;
- FIG. 4 is a schematic sectional view of an organic light-emitting diode (OLED), according to an exemplary embodiment of the present invention with an emission layer and one electron transport layer;
- FIG. 5 is a schematic sectional view of an organic light-emitting diode (OLED), according to an exemplary embodiment of the present invention with an emission layer and two electron transport layers;
- FIG. 6 is a schematic sectional view of an OLED, according to an exemplary embodiment of the present invention with an emission layer and three electron transport layers.

Reference will now be made in detail to the exemplary aspects, examples of which are illustrated in the accompanying drawings, wherein like reference numerals refer to the like elements throughout. The exemplary embodiments are described below, in order to explain the aspects, by referring to the figures.

Herein, when a first element is referred to as being formed or disposed "on" a second element, the first element can be disposed directly on the second element, or one or more other elements may be disposed there between. When a first element is referred to as being formed or disposed "directly on" a second element, no other elements are disposed there between.

The term "contacting sandwiched" refers to an arrangement of three layers whereby the layer in the middle is in direct contact with the two adjacent layers.

The organic light emitting diodes according to an embodiment of the present invention may include a hole transport region; an emission layer; and a first electron transport layer comprising a compound according to formula 1.

FIG. 1 is a schematic sectional view of an organic light-emitting diode 100, according to an exemplary embodiment of the present invention. The OLED 100 comprises an emission layer 150, an electron transport layer (ETL) 161 comprising compound of formula 1 and an electron injection layer 180, whereby the first electron transport layer 161 is disposed directly on the emission layer 150 and the electron injection layer 180 is disposed directly on the first electron transport layer 161.

FIG. 2 is a schematic sectional view of an organic light-emitting diode 100, according to an exemplary embodiment of the present invention. The OLED 100 comprises an emission layer 150 and an electron transport layer stack (ETL) 160 comprising a first electron transport layer (ETL1) 161 comprising compound of formula 1 and a second electron transport layer (ETL2) 162, whereby the second electron transport layer 162 is disposed directly on the first electron transport layer 161. Alternatively, the electron transport layer stack (ETL) 160 comprises a first electron transport layer 161 and a second electron transport layer 162 comprising a compound of formula 1, whereby the second electron transport layer 162 is disposed directly on the first electron transport layer 161.

FIG. 3 is a schematic sectional view of an organic light-emitting diode 100, according to an exemplary embodiment of the present invention. The OLED 100 comprises an emission layer 150 and an electron transport layer stack (ETL) 160 comprising a first electron transport layer (ETL1) 161 that comprises compound of formula 1, a second electron transport layer (ETL2) 162 that comprises compound of formula 1 but different to the compound of the first electron transport layer, and a third electron transport layer (ETL3) 163, whereby the second electron transport layer 162 is disposed directly on the first electron transport layer 161 and the third electron transport layer 163 is disposed directly on the first electron transport layer 162.

FIG. 4 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, one first electron transport layer (ETL1) 161, an electron injection layer (EIL) 180, and a cathode electrode 190. The first electron transport layer (ETL1) 161 comprises compound of formula 1 and optionally an alkali halide or alkali organic complex. The electron transport layer (ETL1) 161 is formed directly on the EML 150.

FIG. 5 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a cathode electrode 190. The electron transport layer (ETL) 160 comprises a first electron transport layer 161 and a second electron transport layer 162, wherein the first electron transport layer is arranged near to the anode (120) and the second electron transport layer is arranged near to the cathode (190). The first and/or the second electron transport layer comprise compound of formula 1 and optionally an alkali halide or alkali organic complex.

FIG. 6 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a second cathode electrode 190. The electron transport layer stack (ETL) 160 comprises a first electron transport layer (ETL1) 161, a second electron transport layer (ETL2) 162 and a third electron transport layer (ETL3) 163. The first electron transport layer 161 is formed directly on the emission layer (EML) 150. The first, second and/or third electron transport layer comprise compound of formula 1 that is different for each layer, and optionally an alkali halide or alkali organic complex.

## Organic semiconductor layer

According to another aspect an organic semiconductor layer may comprise at least one compound of formula 1 and/or formula 1a.

According to one embodiment the organic semiconductor layer may comprises at least one compound of formula 1 and further comprises a metal, metal salt or organic alkali metal complex, preferably alkali metal complex, more preferably LiQ or alkali borate.

According to one embodiment the organic semiconductor layer may comprises at least one compound of formula 1 and further comprises a metal, metal salt or organic metal complex, preferably an organic monovalent or divalent metal complex, more preferably LiQ or alkali borate.

According to one embodiment the organic semiconductor layer may comprises at least one compound of formula 1 and LiQ.

According to one embodiment the organic semiconductor layer may comprises at least one compound of formula 1 and alkali borate.

According to one embodiment, wherein at least one organic semiconductor layer is arranged between the emission layer and the cathode, preferably between the electron injection layer and the cathode.

In another embodiment, the organic semiconductor layer is a first electron transport layer and it is arranged between the emission layer and the second electron transport layer.

According to one embodiment, the organic semiconductor layer is arranged between the first and second emission layer. The organic semiconductor layer can be an electron transport layer, an emission layer, a hole blocking layer, a charge generation layer and/or an electron injection layer, preferably an electron transport layer or a charge generation layer, and more preferred an electron transport layer.

According to one embodiment, the organic semiconductor layer can be arranged between a photoactive layer and a cathode layer, preferably between an emission layer or light-absorbing layer and the cathode layer, preferably the organic semiconductor layer is an electron transport layer.

According to one embodiment, the organic semiconductor layer may comprise at least one alkali halide or alkali organic complex.

An organic semiconductor layer comprises a compound according to formula 1 or 1a is essentially non-emissive or non-emitting.

## Organic electronic device

An organic electronic device according to the invention comprises at least one organic semiconductor layer, wherein at least one organic semiconductor layer comprises a compound according to formula 1.

An organic electronic device according to one embodiment, which comprises at least one organic semiconductor layer that comprises a compound according to formula 1, wherein this layer is essentially non-emissive or non-emitting.

According to one embodiment, the organic electronic device may comprises at least one organic semiconductor layer comprising compound of formula 1 that is an electron transport layer, an emission layer, a hole blocking layer, a charge generation layer and/or an electron injection layer, preferably an electron transport layer or a charge generation layer, more preferred an electron transport layer.

An organic electronic device according to one embodiment may include a substrate, an anode layer, an organic

semiconductor layer comprising compound of formula 1, and a cathode layer.

The organic electronic device according to according to one embodiment may comprises at least one organic semiconductor layer, wherein the organic semiconductor layer comprising compound of formula 1 is arranged between a photoactive layer and a cathode layer, preferably between an emission layer or light-absorbing layer and the cathode layer, preferably the organic semiconductor layer is an electron transport layer

The organic electronic device according to one embodiment may comprises at least one organic semiconductor layer comprising compound of formula 1, wherein the at least one organic semiconductor layer further comprises at least one alkali halide or alkali organic complex.

An organic electronic device according to one embodiment comprises at least one organic semiconductor layer comprising at least one compound of formula 1, at least one anode layer, at least one cathode layer and at least one emission layer, wherein the organic semiconductor layer comprising at least one compound of formula 1 is preferably arranged between the emission layer and the cathode layer.

An organic electronic device according to one embodiment comprises at least one organic semiconductor layer comprising at least one compound of formula 1 and further comprises at least one alkali halide or alkali organic complex.

An organic electronic device according to one embodiment comprises at least one organic semiconductor layer, at least one anode layer, at least one cathode layer and at least one emission layer, wherein the organic semiconductor layer comprising at least one compound of formula 1 is preferably arranged between the emission layer and the cathode layer. Preferably the at least one organic semiconductor layer is an electron transport layer.

An organic light-emitting diode (OLED) according to the invention may include an anode, a hole transport layer (HTL), an emission layer (EML), an electron transport layer (ETL) comprising at least one compound of formula 1, and a cathode, which are sequentially stacked on a substrate. In this regard, the HTL, the EML, and the ETL are thin films formed from organic compounds.

An organic electronic device according to one embodiment can be a light emitting device, thin film transistor, a battery, a display device or a photovoltaic cell, and preferably a light emitting device. A light emitting device can be an OLED.

According to one embodiment the OLED may have the following layer structure, wherein the layers having the following order:

an anode layer, a hole injection layer, optional a first hole transport layer, optional a second hole transport layer, an emission layer, an electron transport layer comprising compound of formula 1 according to the invention, an electron injection layer, and a cathode layer.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of formula 1 according to the invention, and
- a second deposition source to release the alkali halide or alkali organic complex, preferably a lithium halide or lithium organic complex;

the method comprising the steps of forming the electron transport layer stack; whereby for an organic light-emitting diode (OLED):

- the first electron transport layer is formed by releasing the compound of formula 1 according to the invention from the first deposition source and the alkali halide or alkali organic complex, preferably a lithium halide or lithium organic complex from the second deposition source.

According to various embodiments of the present invention, the method may further include forming on the

anode electrode an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, preferably a first electron transport layer is formed on the emission layer and a second electron transport layer is formed on the first electron transport layer and the second electron transport layer comprises a compound of formula 1,
- and finally a cathode electrode is formed,
- optional a hole injection layer, a hole transport layer, and a hole blocking layer, formed in that order between the first anode electrode and the emission layer,
- optional an electron injection layer is formed between the electron transport layer stack and the cathode electrode.

According to various embodiments of the present invention, the method may further include forming an electron injection layer on a first electron transport layer. However, according to various embodiments of the OLED of the present invention, the OLED may not comprise an electron injection layer.

According to various embodiments, the OLED may have the following layer structure, wherein the layers having the following order:

an anode, first hole transport layer, second hole transport layer, emission layer, optional second electron transport layer, first electron transport layer comprising compound of formula 1 according to the invention, optional a second electron transport layer, optional an electron injection layer, and a cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

Preparation of compounds of formula 1

Compounds of formula 1 may be prepared as described in WO2016171358, WO2017135510 and below.

Preparation of compound of formula 1

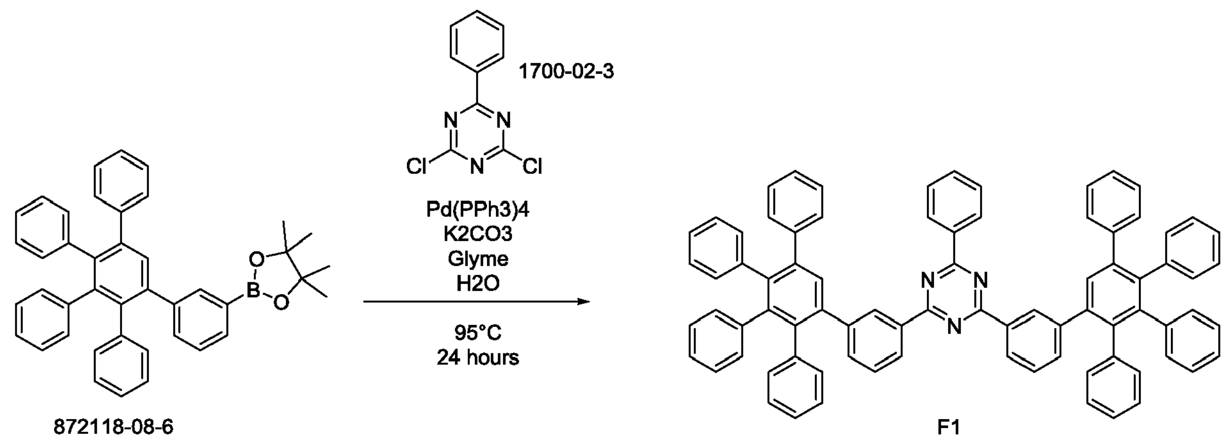


872118-08-6

F1

4,4,5,5-tetramethyl-2-(3',4',5'-triphenyl-[1,1':2',1''-terphenyl]-3-yl)-1,3,2-dioxaborolane (15.0 g, 25.7 mmol, 2.4 eq., CAS 872118-08-6), 2,4-dichloro-6-phenyl-1,3,5-triazine (2.4 g, 10.7 mmol, 1.0 eq., CAS 1700-02-3), potassium

carbonate (665 mg, 48,1 mmol, 4.5 eq.) were placed in a flask under a nitrogen stream. Desoxygenated glyme (100 mL) and desoxygenated water (25 mL) were added and the reaction mixture was flushed with nitrogen. Then it was stirred under nitrogen overnight at 95 °C. The reaction mixture was cooled down, precipitate was filtered and washed with glyme (2 x 10 mL), water (4 x 10 mL) and glyme (2 x 10 mL). Crude compound was dissolved in chloroform (300 mL) and filtered over a pad of silicagel (6.0cm x 2.0cm) using chloroform (100 mL) as eluent. Solvent was partially removed under vacuum, then hexane (30 mL) was added to induce precipitation. Solid was filtered to obtain 3.4 g (30% yield) of 2-phenyl-4,6-bis(3',4',5'-triphenyl-[1,1':2',1"-terphenyl]-3-yl)-1,3,5-triazine.

Compounds of formula 1 with two $X^1$, $X^2$, $X^3$ selected from N and one $X^1$, $X^2$, $X^3$ selected from C may be prepared as shown in the reaction scheme above, wherein 4-chloro-2,6-diphenylpyrimidine or 2-chloro-4,6-diphenylpyrimidine is used instead of 2-chloro-4,6-diphenyl-1,3,5-triazine.

The chemical structure, molar mass, calculated HOMO, LUMO, dipole moment, melting point, glass transition temperature and rate onset temperature of compounds of formula 1 of example 1 and comparative examples 1 to 3 are shown in Table 3.

General procedure for fabrication of organic electronic devices

Electron-only devices

For electron-only devices (EOD), see Table 3, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes. 100 nm Ag were deposited as anode on the glass substrate at a pressure of $10^{-5}$ to $10^{-7}$ mbar.

Then, LiQ was deposited on the Ag layer to form a layer with a thickness of 1 nm.

Then, 50 vol.-% compound G1, G3 or F1 and 50 vol.-% LiQ is deposited on the LiQ layer to form an organic semiconductor layer with a thickness of 36 nm, see comparative examples 1 and 3 and example 1 in Table 3.

Then, 70 vol.% 3-Phenyl-3H-benzo[b]dinaphtho[2,1-d:1',2'-f]phosphepine-3-oxide and 30 vol.-% Lithium tetra(1H-pyrazol-1-yl)borate were deposited to form an electron injection layer with a thickness of 3 nm.

Then, MgAg alloy (90:10 vol.-%) was deposited on the electron injection layer to form a cathode electrode with a thickness of 30 nm.

The electron-only device is protected from ambient conditions by encapsulation of the device with a glass slide. Thereby, a cavity is formed, which includes a getter material for further protection.

The operating voltage was determined at 10 mA/cm$^2$, see Table 3.

General procedure for fabrication of OLEDs

For top emission devices, Example 2 and Comparative Example 4, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes. 100 nm Ag were deposited on the glass substrate as anode at a pressure of $10^{-5}$ to $10^{-7}$ mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the ITO electrode, to form a HIL having a thickness of 10 nm. Then, Biphenyl-4-yl(9,9-diphenyl-9H-fluoren-2-yl)-[4-(9-phenyl-9H-carbazol-3-yl)phenyl]-amine was vacuum deposited on the HIL, to form a HTL having a thickness of 118 nm.

Then, N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% ABH113 (Sun Fine Chemicals) as EML host and 3 vol.-% NUBD370 (Sun Fine Chemicals) as fluorescent blue dopant were deposited on the EBL, to form a blue-emitting EML with a thickness of 20 nm.

Then, a first electron transport layer is formed on the EML with a thickness of 5 nm by depositing the compound F1 of formula 1 or compound G2 on the emission layer with a thickness of 5 nm, see Table 4.

Then, the second electron transporting layer is formed on the first electron transport layer according to Example 2 and Comparative Example 4 with a the thickness of 31 nm. The second electron transport layer comprises 50 vol.-% 2-([1,1'-biphenyl]-4-yl)-4-(9,9-diphenyl-9H-fluoren-4-yl)-6-phenyl-1,3,5-triazine, also named compound G4, as matrix compound and 50 vol.-% of alkali organic complex, see Table 4.

Then, the electron injection layer is formed on the electron transporting layer by deposing Yb with a thickness of 2 nm.

Ag is evaporated at a rate of 0.01 to 1 Å/s at $10^{-7}$ mbar to form a cathode with a thickness of 11 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m$^2$ using an Instrument Systems CAS-140CT array spectrometer for each of the voltage values. The cd/A efficiency at 10 mA/cm$^2$ is determined by interpolating the luminance-voltage and current-voltage characteristics, respectively.

Lifetime LT of the device is measured at ambient conditions (20°C) and 30 mA/cm$^2$, using a Keithley 2400 sourcemeter, and recorded in hours.

The brightness of the device is measured using a calibrated photo diode. The lifetime LT is defined as the time till the brightness of the device is reduced to 97 % of its initial value.

The light output in external efficiency EQE and power efficiency (lm/W efficiency) are dertermined at 10 mA/cm$^2$ for top emission devices.

To determine the efficiency EQE in % the light output of the device is measured using a calibrated photodiode.

To determine the power efficiency in lm/W, in a first step the luminance in candela per square meter (cd/m2) is measured with an array spectrometer CAS140 CT from Instrument Systems which has been calibrated by Deutsche Akkreditierungs-stelle (DAkkS). In a second step, the luminance is then multiplied by $\pi$ and divided by the voltage and current density.

Technical Effect of the invention

As can be seen in Table 3, the mp and Tg for F1 is improved over G1 to G3 of the Comparative Examples 1 to 3. Surprisingly, the rate onset temperature $T_{RO}$ of compounds of formula 1 are in a range suitable for use in organic electronic devices, even though the molecular weight is significantly higher than for comparative examples 1 to 3.

Additionally, the operating voltage in electron-only devices is lower for an organic semiconductor layer comprising compound of formula 1 compared to comparative examples 1 and 3. A lower operating voltage may indicate higher conductivity of the organic semiconductor layer. A low operating voltage is beneficial for battery life, in particular of mobile devices.

Top emission devices

In Table 4 is shown the performance of an organic electronic device comprising an organic semiconductor layer comprising a compound F1 of formula 1 and an alkali organic complex.

In Comparative Example 4, the first electron transport layer (ETL1) comprises compound G2. The second electron transport layer (ETL2) comprises 50 vol.-% compound G4 and 50 vol.-% LiQ. The operating voltage is 3.7 V and the cd/A efficiency is 7.6 cd/A. The lifetime is 9 hours.

In Example 2, the first electron transport layer (ETL1) comprises compound F1 of formula 1. The second electron transport layer (ETL2) comprises 50 vol.-% compound G4 and 50 vol.-% LiQ. The operating voltage is 3.7 V. The cd/A efficiency is improved to 7.9 cd/A and the lifetime is improved to 13 hours.

In summary, improved cd/A efficiency and/or lifetime may be achieved when the organic semiconductor layer comprises a compound of formula 1.

Table 3: Properties of comparative examples 1 to 3 and compound F1 of formula 1

| Referred to as: | Structure | Molar mass (g/mol) | Calculated HOMO (eV) | Calculated LUMO (eV) | Dipole moment (Debye) | mp (°C) | Tg (°C) | $T_{RO}$ (°C) | Operating voltage in EOD (V) |
|---|---|---|---|---|---|---|---|---|---|
| Comparative example 1 G1 | 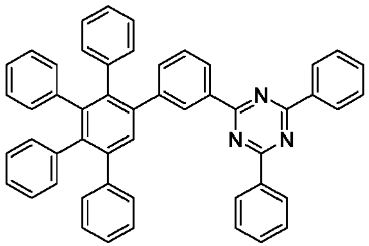 | 689.84 | -5.82 | -1.76 | 0.57 | 280 | 138 | 211 | 0.5 |
| Comparative example 2 G2 | 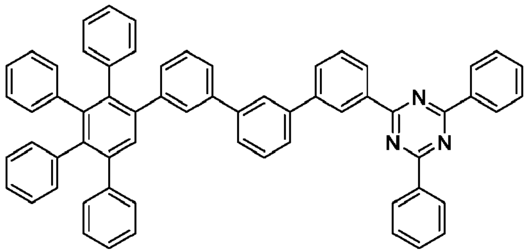 | 842.04 | -5.72 | -1.82 | 0.30 | - | 141 | 267 | 0.6 |
| Comparative example 3 G3 | 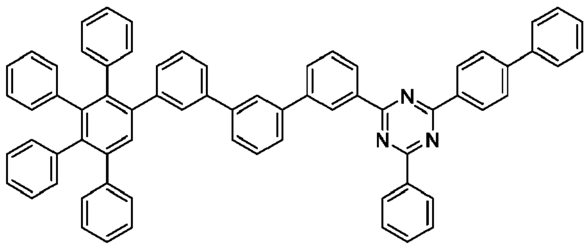 | 918.13 | -5.76 | -1.94 | 1.23 | 302 | 149 | 292 | - |
| Example 1 F1 | 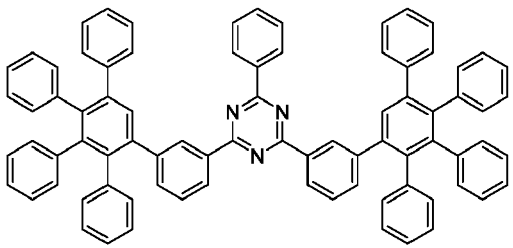 | 1070.32 | -5.75 | -1.76 | 0.89 | 322 | 182 | 266 | 0.35 |

Table 4: Performance of an organic electroluminescent device comprising a first electron transport layer comprising the compound F1 of formula 1

| | Matrix compound in ETL1 | Thickness ETL1 (nm) | Matrix compound in ETL2 | Concentration of matrix compound in ETL2 (vol.-%) | Alkali organic complex in ETL2 | Concentration of alkali organic complex in ETL2 (vol.-%) | Thickness ETL2 (nm) | Operating voltage at 10 $mA/cm^2$ (V) |
|---|---|---|---|---|---|---|---|---|
| Comparative example 4 | G2 | 5 | G4 | 50 | LiQ | 50 | 31 | 3.7 |
| Example 2 | F1 | 5 | G4 | 50 | LiQ | 50 | 31 | 3.7 |

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims. Therefore, the aforementioned embodiments should be understood to be exemplary but not limiting the present invention in any way.

## Claims

1. A compound according to formula 1:

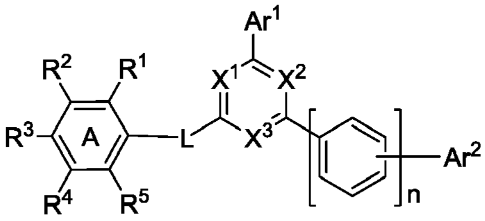

(1),

wherein

$X^1$ to $X^3$ are selected from N or C, with the proviso that at least two of $X^1$ to $X^3$ are selected from N;
L is phenylene;
$R^1$ to $R^5$ are independently selected from H, phenyl or pyridyl, with the proviso that at least 2 of $R^1$ to $R^5$ is phenyl or pyridyl;
$Ar^1$ is selected from substituted or unsubstituted $C_6$ to $C_{18}$ aryl or substituted or unsubstituted $C_2$ to $C_{20}$ heteroaryl;
$Ar^2$ is selected from substituted or unsubstituted aryl group comprising at least 3 phenyl rings; and
n is selected from 0, 1 or 2;

wherein L and at least one of $R^1$ and $R^5$, which are phenyl or pyridyl, are arranged in ortho-position to each other on ring A and/or wherein at least two of $R^1$ to $R^5$, which are phenyl or pyridyl, are arranged in ortho-position to each other on ring A.

2. The compound of formula 1 according to claim 1, wherein $X^1$ to $X^3$ are N.

3. The compound of formula 1 according to claim 1 or 2, wherein at least 3 to 5 of $R^1$ to $R^5$ are phenyl or pyridyl, preferably at least 3 to 5 of $R^1$ to $R^5$ are phenyl.

4. The compound of formula 1 according to any of claims 1 to 3, wherein $Ar^2$ is selected from substituted or unsubstituted aryl group comprising at least 3 substituted or unsubstituted phenyl rings to 12 substituted or unsubstituted phenyl rings, preferably 4 substituted or unsubstituted phenyl rings to 10 substituted or unsubstituted phenyl rings, and further preferred 4 substituted or unsubstituted phenyl rings to 6 substituted or unsubstituted phenyl rings, wherein the substituents of the substituted aryl group are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl; and preferably $Ar^2$ is selected from unsubstituted $C_6$ to $C_{18}$ aryl.

5. The compound of formula 1 according to any of claims 1 to 4, wherein

- for n = 1, formula 1 has the structure 2:

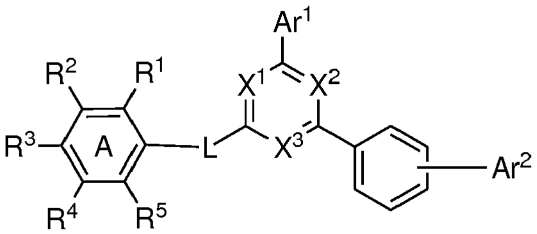

(2);

- for n = 0, formula 1 has the structure 3:

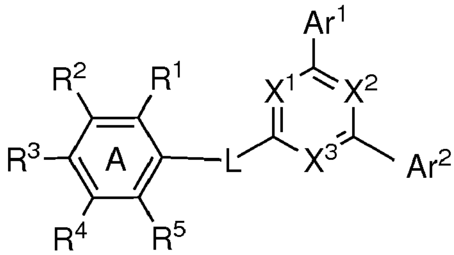

(3).

6. The compound of formula 1 according to any of claims 1 or 5, wherein

$Ar^1$ is selected from substituted or unsubstituted $C_6$ to $C_{18}$ aryl, wherein the substituents of the substituted $C_6$ to $C_{18}$ aryl are selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl; and
preferably $Ar^1$ is selected from unsubstituted $C_6$ to $C_{18}$ aryl.

7. The compound of formula 1 according to any of claims 1 to 6, wherein
$R^1$ and $R^2$ are phenyl or pyridyl, or
$R^1$ and $R^3$ are phenyl or pyridyl, or
$R^2$ and $R^3$ are phenyl or pyridyl, or
$R^1$, $R^2$ and $R^3$ are phenyl or pyridyl, or
$R^1$, $R^2$, $R^3$ and $R^4$ are phenyl or pyridyl, or
$R^1$, $R^2$, $R^3$, $R^4$ and $R^5$ are phenyl or pyridyl, or
$R^2$, $R^3$ and $R^4$ are phenyl or pyridyl;
preferably $R^1$ and/or $R^3$ are phenyl or pyridyl, further preferred $R^3$ is phenyl or pyridyl, also preferred $R^1$ and $R^3$ are phenyl or pyridyl.

8. The compound of formula 1 according to any of claims 1 to 7, wherein $Ar^2$ is independently selected from B1 to B19:

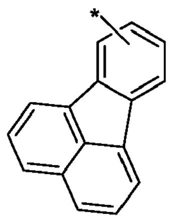

(B1),

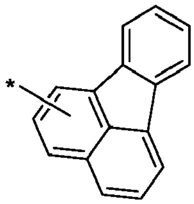

(B2),

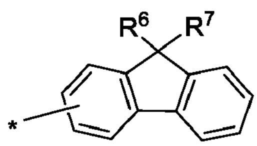

(B3),

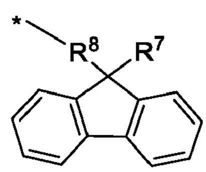

(B4),

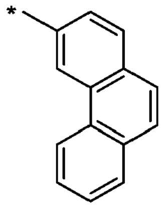

(B5),

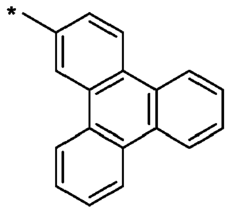

(B6),

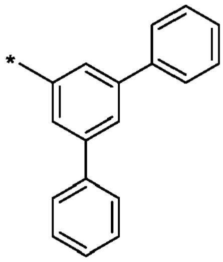

(B7),

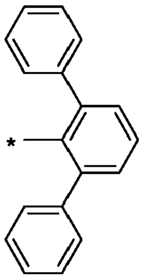

(B8),

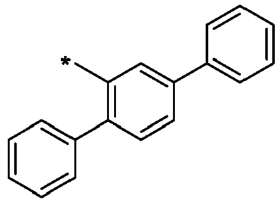

(B9),

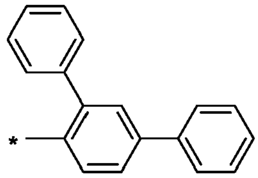

(B10),

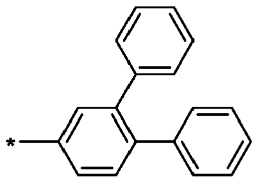

(B11),

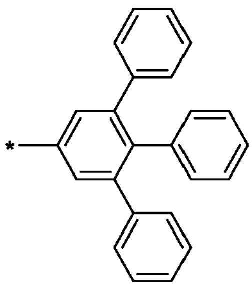

(B12),

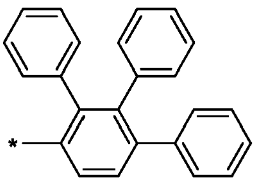

(B13),

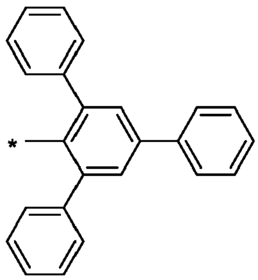

(B14),

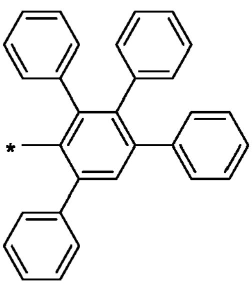

(B15),

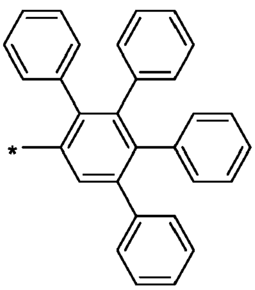

(B16),

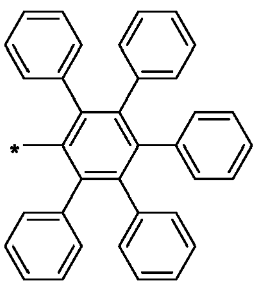

(B17),

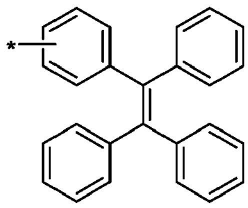 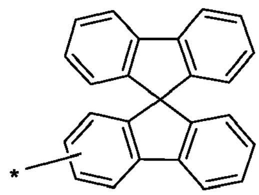

(B18), (B19);

wherein

$R^6$ and $R^7$ are independently selected from H, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy or $C_6$ to $C_{24}$ aryl;
$R^8$ is selected from $C_6$ to $C_{18}$ arylene.

**9.** The compound of formula 1 according to any of claims 1 to 8, wherein the compound of formula 1 is selected from C1 to C4:

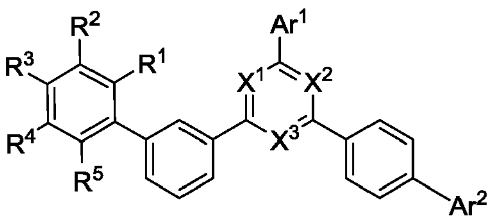 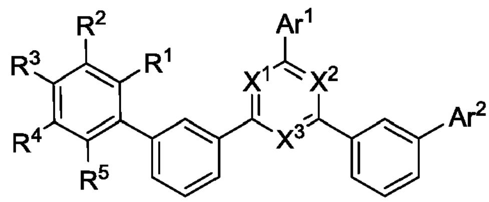

(C1), (C2),

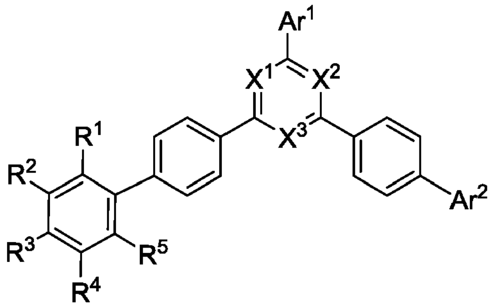 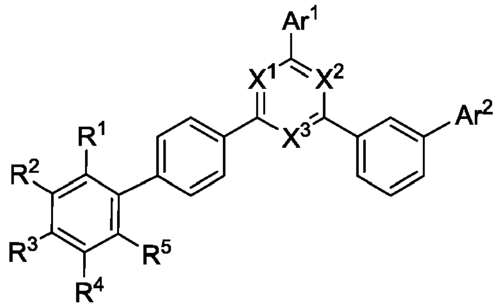

(C3), (C4),

wherein

$X^1$ to $X^3$ are selected from N or C, with the proviso that at least two of $X^1$ to $X^3$ are selected from N;
$R^1$ to $R^5$ are independently selected from H, phenyl or pyridyl, with the proviso that at least two of $R^1$ to $R^5$ are phenyl or pyridyl; and at least two of $R^1$ to $R^5$ that are phenyl or pyridyl;
$Ar^1$ is selected from substituted or unsubstituted $C_6$ to $C_{18}$ aryl or substituted or unsubstituted $C_2$ to $C_{20}$ heteroaryl, preferably phenyl, wherein the substituents of the substituted aryl and substituted heteroaryl are independently selected from $C_1$ to $C_{12}$ alkyl, $C_1$ to $C_{12}$ alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{18}$ aryl, $C_3$ to $C_{25}$ heteroaryl, D, F or CN, preferably from $C_1$ to $C_{12}$ alkyl;
$Ar^2$ is selected from substituted or unsubstituted aryl group comprising at least three phenyl rings, preferably three to four phenyl rings.

**10.** The compound of formula 1 according to any of claims 1 to 9, wherein

- n is 1; two of $X^1$, $X^2$ and $X^3$ are N and one is C, preferably $X^1$ and $X^2$ are N and $X^3$ is C; $Ar^1$ is phenyl; $R^1$, $R^2$, $R^3$, $R^4$, $R^5$ are independently selected from H and phenyl; and $Ar^2$ is selected from B1 to B19; with the proviso that at least 2 of $R^1$ to $R^5$ are phenyl, preferably at least 3 of $R^1$ to $R^5$ are phenyl, and in addition preferred 4 of $R^1$ to $R^5$ are phenyl and one is H; or
- n is 1; $X^1$, $X^2$ and $X^3$ are N; $Ar^1$ is phenyl; $R^1$, $R^2$, $R^3$, $R^4$, $R^5$ are independently selected from H and phenyl and $Ar^2$ is selected from B1 to B19; with the proviso that at least 2 of $R^1$ to $R^5$ are phenyl, preferably at least 3 of $R^1$ to $R^5$ are phenyl, and in addition preferred 4 of $R^1$ to $R^5$ are phenyl and one is H.

**11.** The compound of formula 1 according to any of claims 1 to 10, wherein the compound of formula 1 is selected from F1:

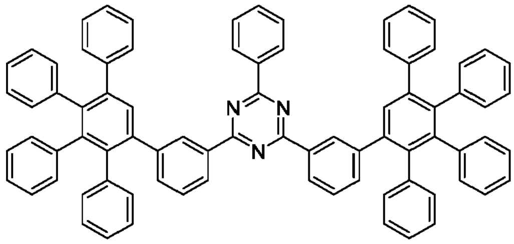

(F1).

**12.** An organic semiconductor layer comprising at least one compound of formula 1 according to any of the preceding claims 1 to 11.

**13.** The organic semiconductor layer according to claim 12, further comprises a metal, metal salt or organic metal complex, preferably an organic monovalent or divalent metal complex, more preferably LiQ or alkali borate.

**14.** An organic electronic device comprising an organic semiconductor layer according to any of the preceding claim 12 or 13, wherein at least one organic semiconductor layer comprises a compound of formula 1 according to any of the preceding claims 1 to 11.

**15.** The organic electronic device according to claim 14, wherein the electronic device is a light emitting device, thin film transistor, a battery, a display device or a photovoltaic cell, and preferably a light emitting device.

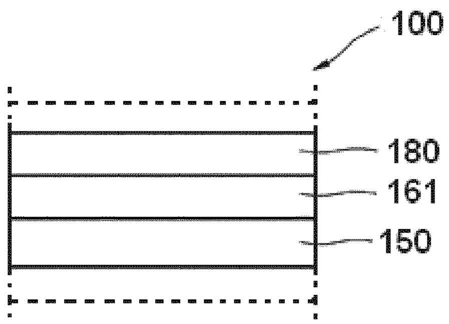


FIG. 1

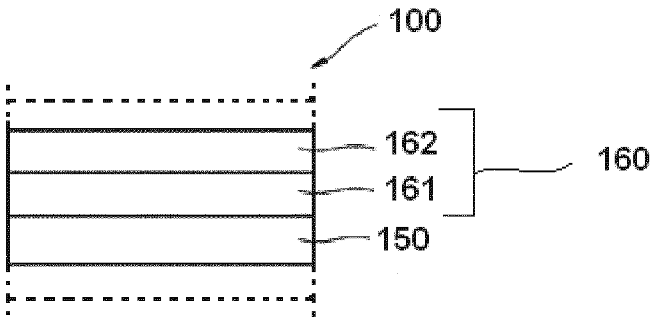


FIG. 2

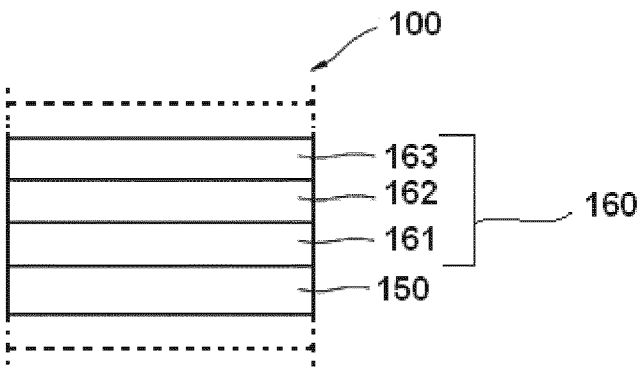


FIG. 3

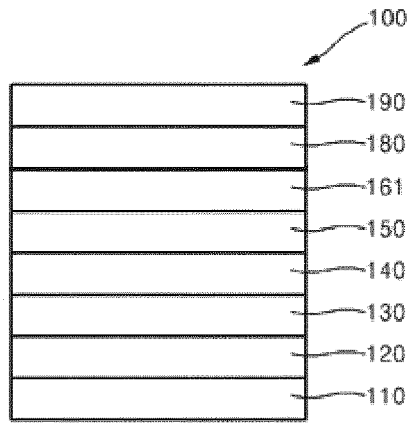


FIG. 4

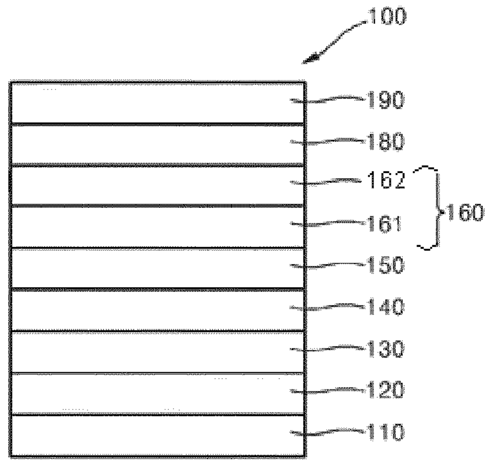


FIG. 5

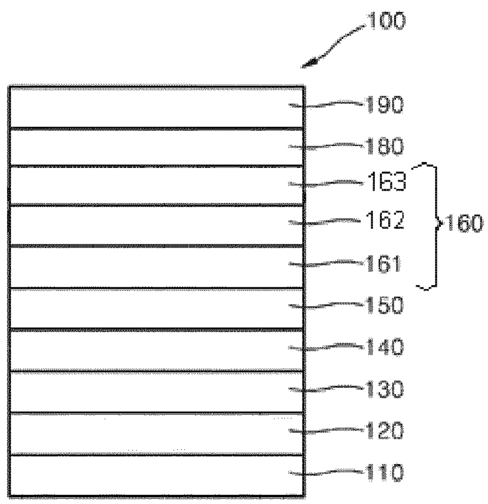


FIG. 6

Europäisches Patentamt
European Patent Office
Office européen des brevets

# EUROPEAN SEARCH REPORT

Application Number
EP 18 15 7180

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | DE 10 2010 054316 A1 (MERCK PATENT GMBH [DE]) 14 June 2012 (2012-06-14)<br>* abstract *<br>* pages 47-48; compounds 352, 358 *<br>* paragraphs [0011], [0066] - [0133] *<br>* examples 1-6 *<br>* claims 1-15 *<br>----- | 1-8, 12-15<br>9-11 | INV.<br>C07D251/24<br>H01L51/00 |
| X<br>Y | WO 2017/135510 A1 (SAMSUNG SDI CO LTD [KR]) 10 August 2017 (2017-08-10)<br>* abstract *<br>* pages 14-17; compounds 5, 13, 21, 29, 37, 45, 53, 61, 69, 77, 85 *<br>* pages 17-18; compounds 93, 101, 109, 145 *<br>* claims 1-15 *<br>----- | 1-8, 12-15<br>9-11 | |
| Y | WO 2017/171420 A1 (LG CHEMICAL LTD [KR]) 5 October 2017 (2017-10-05)<br>* abstract *<br>* examples 1-26 *<br>* claims 1-10 *<br>----- | 1-15 | TECHNICAL FIELDS SEARCHED (IPC) |
| Y | US 2017/200902 A1 (LEE SEUNG-JAE [KR] ET AL) 13 July 2017 (2017-07-13)<br>* abstract *<br>* paragraphs [0010] - [0026] *<br>* compounds *;<br>pages 7-24<br>* claims 1-14 *<br>----- | 1-15 | C07D<br>H01L |
| Y | EP 3 127 988 A1 (LG CHEMICAL LTD [KR]) 8 February 2017 (2017-02-08)<br>* abstract *<br>* paragraphs [0064], [0309] *<br>* paragraph [0193] *<br>* claims 1-32 *<br>-----<br>-/-- | 1-15 | |

The present search report has been drawn up for all claims

1

| Place of search | Date of completion of the search | Examiner |
|---|---|---|
| Munich | 17 May 2018 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 15 7180

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/349269 A1 (LEE HAN-ILL [KR] ET AL) 3 December 2015 (2015-12-03)<br>* abstract *<br>* compounds *; pages 7-22<br>* claims 1-14 *<br>----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 17 May 2018 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 18 15 7180

This annex lists the patent family members relating to the patent documents cited in the above-mentioned European search report. The members are as contained in the European Patent Office EDP file on
The European Patent Office is in no way liable for these particulars which are merely given for the purpose of information.

17-05-2018

| Patent document cited in search report | Publication date | Patent family member(s) | Publication date |
|---|---|---|---|
| DE 102010054316 A1 | 14-06-2012 | CN 103261131 A | 21-08-2013 |
| | | CN 105732294 A | 06-07-2016 |
| | | DE 102010054316 A1 | 14-06-2012 |
| | | EP 2651862 A1 | 23-10-2013 |
| | | JP 6271252 B2 | 31-01-2018 |
| | | JP 2014508114 A | 03-04-2014 |
| | | JP 2016196460 A | 24-11-2016 |
| | | KR 20130127994 A | 25-11-2013 |
| | | US 2013261708 A1 | 03-10-2013 |
| | | US 2015057445 A1 | 26-02-2015 |
| | | WO 2012079678 A1 | 21-06-2012 |
| WO 2017135510 A1 | 10-08-2017 | KR 20170093023 A | 14-08-2017 |
| | | TW 201728574 A | 16-08-2017 |
| | | WO 2017135510 A1 | 10-08-2017 |
| WO 2017171420 A1 | 05-10-2017 | KR 20170113397 A | 12-10-2017 |
| | | WO 2017171420 A1 | 05-10-2017 |
| US 2017200902 A1 | 13-07-2017 | CN 106470978 A | 01-03-2017 |
| | | KR 20160019747 A | 22-02-2016 |
| | | TW 201605815 A | 16-02-2016 |
| | | US 2017200902 A1 | 13-07-2017 |
| | | WO 2016024745 A2 | 18-02-2016 |
| EP 3127988 A1 | 08-02-2017 | CN 106164215 A | 23-11-2016 |
| | | EP 3127988 A1 | 08-02-2017 |
| | | JP 2017513224 A | 25-05-2017 |
| | | KR 101537500 B1 | 20-07-2015 |
| | | KR 20150115649 A | 14-10-2015 |
| | | KR 20150115688 A | 14-10-2015 |
| | | TW 201600513 A | 01-01-2016 |
| | | WO 2015152634 A1 | 08-10-2015 |
| US 2015349269 A1 | 03-12-2015 | CN 105073718 A | 18-11-2015 |
| | | EP 2998301 A1 | 23-03-2016 |
| | | JP 6257113 B2 | 10-01-2018 |
| | | JP 2016526031 A | 01-09-2016 |
| | | KR 20140135524 A | 26-11-2014 |
| | | TW 201444952 A | 01-12-2014 |
| | | US 2015349269 A1 | 03-12-2015 |
| | | WO 2014185598 A1 | 20-11-2014 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6140763 A
- US 6614176 B
- US 2016248022 A
- WO 2013079217 A1
- WO 2013079676 A1
- WO 2013079678 A1
- JP 2008195623 B
- JP 2001291593 B
- US 20030165711 A
- WO 2016171358 A
- WO 2017135510 A